# EUROPEAN PATENT APPLICATION

(11) **EP 4 378 944 A1**
(43) Date of publication of application: **05.06.2024**
(21) Application number: 22876499.9
(22) Date of filing: 29.09.2022
(51) Int. Cl.: C07D 495/04, C07K 7/06, C07K 7/08, C07K 17/00, C07F 9/6524

(54) **COMPOUND HAVING PHOTOCLEAVABLE SITE, LINKER, AND SCREENING METHOD USING SAME**

(30) Priority: 30.09.2021 JP 2021161962
(71) Applicant: CHUGAI SEIYAKU KABUSHIKI KAISHA, Tokyo 115-8543 (JP)
(72) Inventor: SAITO, Rie, Kamakura-shi, Kanagawa 247-8530 (JP); NAKANO, Kazuhiko, Kamakura-shi, Kanagawa 247-8530 (JP); OHTA, Atsushi, Kamakura-shi, Kanagawa 247-8530 (JP); EMURA, Takashi, Gotemba-shi, Shizuoka 412-8513 (JP)
(74) Representative: Vossius & Partner Patentanwälte Rechtsanwälte mbB
(86) International application number: PCT/JP2022/036570
(87) International publication number: WO 2023/054636

(57) **Abstract**

The present invention relates to a compound having (i) a photocleavable moiety, and (ii) at least one selected from the group consisting of a target substance binding site and an anchor binding site.

## Description

### [Technical Field]

The present invention relates to a compound and a conjugate. The present invention also relates to a method for selecting a substance of interest capable of binding to a target substance.

### [Background Art]

Conventionally, a method of repeating operations of bringing into contact a library of peptide compounds with an immobilized target substance, eluting the molecules bound to the target substance, and then amplifying the eluted molecules is known as a method for selecting from a library and enriching peptide compounds with binding selectivity for a target substance. Such operations are called panning. In conventional panning, heat elution or protease has been used to elute the molecules bound to the target substance (e.g., Patent Literature 1).

### [Citation List]

### [Patent Literature]

[Patent Literature 1] International Publication No. WO 2013/100132

### [Summary of Invention]

### [Technical Problem]

When elution by heat was used, the efficiency of enrichment was not always sufficient due to other peptide compounds than those bound to the target substance also being recovered. In addition, the present inventors have conducted studies and found that even when using proteases, there were various problems, such as the efficiency of elution varying depending on the target substance, other peptide compounds than those bound to the target substance being recovered due to the need for heating, and the polymerase chain reaction (PCR), which is the step post-elution, being inhibited, and therefore, while superior to heat elution, the efficiency of enrichment was still not sufficient.

An object of the present invention is to provide a compound or intermediate thereof which can be utilized for a novel panning that uses light for elution.

### [Solution to Problem]

The present invention relates to, for example, each of the following inventions.
[1] A compound, having:
   (i) a photocleavable moiety; and
   (ii) at least one selected from the group consisting of a target substance binding site and an anchor binding site.
[2] The compound according to [1], wherein the target substance binding site is an enzyme recognition site.
[3] The compound according to [1] or [2], having:
   (i) a photocleavable moiety;
   (ii-1) a target substance binding site; and
   (ii-2) an anchor binding site.
[4] The compound according to any one of [1] to [3], having a plurality of the photocleavable moieties.
[5] The compound according to any one of [1] to [4], having 2 or more and 5 or less of the photocleavable moieties.
[6] The compound according to either [4] or [5], wherein the plurality of photocleavable moieties have all the same structure.
[7] The compound according to any one of [1] to [6], wherein the target substance binding site is recognized by at least one selected from the group consisting of ligases and proteases.
[8] The compound according to any one of [1] to [7], wherein the target substance binding site comprises an amino acid residue.
[9] The compound according to any one of [1] to [8], wherein the target substance binding site comprises two or more amino acid residues.
[10] The compound according to any one of [1] to [9], wherein the target substance binding site comprises a glycine residue.
[11] The compound according to any one of [1] to [10], wherein the amino acid residue in the target substance binding site is protected with a protective group.
[12] The compound according to [11], wherein the protective group is at least one selected from the group consisting of a 9-fluorenylmethyloxycarbonyl group, a tert-butoxycarbonyl group, a benzyloxycarbonyl group, a 2-nitrobenzenesulfonyl group and an allyloxycarbonyl group.
[13] The compound according to any one of [1] to [12], wherein the absorption wavelength at which the photocleavable moiety undergoes cleavage is in the range of 300 nm or more and 500 nm or less.
[14] The compound according to any one of [1] to [13], wherein the absorption wavelength at which the photocleavable moiety undergoes cleavage is a wavelength in the UV-A region.
[15] The compound according to any one of [1] to [14], wherein the photocleavable moiety has a nitroveratryloxycarbonyl residue or a coumarin residue.
[16] The compound according to any one of [1] to [15], wherein the photocleavable moiety has a structure represented by the following formula (1): 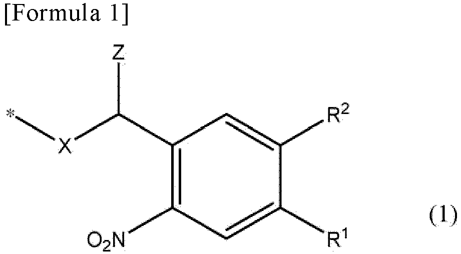 wherein
   X represents O or NH;
   Z represents a hydrogen atom or a C1-6 alkyl group;
   R¹ represents -O-L¹¹-L¹²-*, a C1-10 alkoxy group, or a hydrogen atom;
   R² represents -O-L¹¹-L¹²-* or a C1-10 alkoxy group;
   L¹¹ represents a C1-10 alkylene group;
   L¹² represents a divalent group selected from the group consisting of a single bond, a C1-10 alkylene group, an amide bond, an ether bond, and combinations thereof;
   any one of R¹ and R² is -O-L¹¹-L¹²-*; and
   * represents a bond.
[17] The compound according to any one of [1] to [15], wherein the photocleavable moiety has a structure represented by the following formula (2): 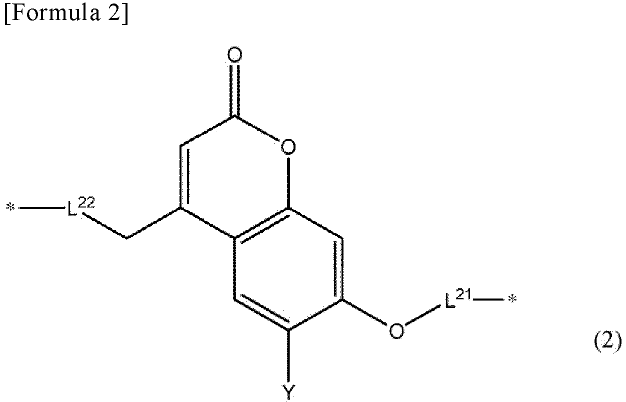 wherein
   Y represents a hydrogen atom or halogen atom;
   L²¹ and L²² each independently represent a divalent group selected from the group consisting of a single bond, a C1-10 alkylene group, an amide bond, an ether bond, and combinations thereof; and
   * represents a bond.
[18] The compound according to any one of [1] to [17], having a plurality of the photocleavable moieties and a spacer arm, and
   wherein some or all of the plurality of photocleavable moieties are bound via the spacer arm.
[19] The compound according to [18], wherein the spacer arm comprises an oxyethylene group, an amino acid residue, or a substituted or unsubstituted alkylene group.
[20] The compound according to [18] or [19], wherein the spacer arm comprises a C1-10 alkylene group.
[21] The compound according to any one of [18] to [20], wherein the spacer arm comprises an ether group.
[22] The compound according to any one of [18] to [21], wherein the spacer arm comprises one or more selected from the group consisting of a polyethylene glycol residue, a polyamine residue, a glycine residue, a serine residue and a threonine residue.
[23] The compound according to any one of [1] to [22], wherein the anchor binding site is a biotin tag.
[24] The compound according to any one of [1] to [23], wherein the anchor binding site is represented by the following formula (C): 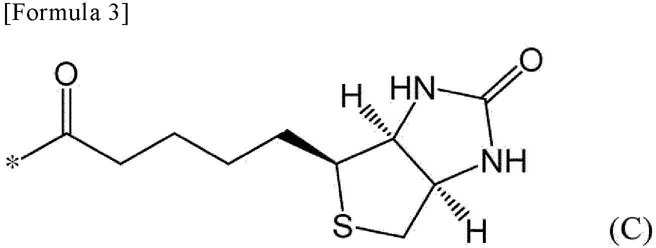
[25] The compound according to any one of [1] to [24], wherein the carrier to which the anchor binding site binds is a solid phase.
[26] The compound according to [1] or [2], wherein the compound is represented by the following formula (3): 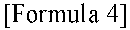 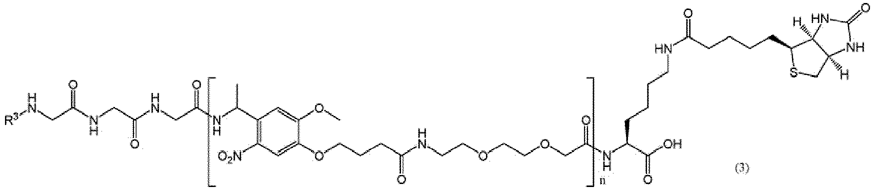 wherein
   n represents an integer of 1 or larger and 5 or smaller, and
   R³ represents a hydrogen atom or a protective group.
[27] The compound according to [1] or [2], wherein the compound is represented by the following formula (4): 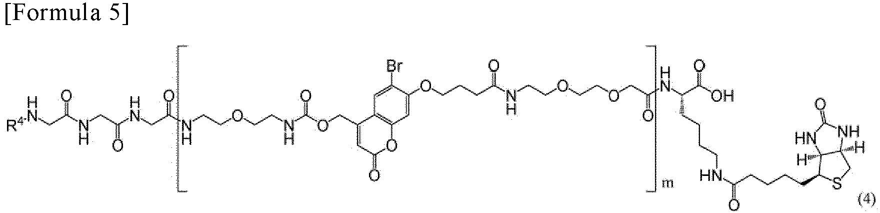 wherein
   m represents an integer of 1 or larger and 5 or smaller, and
   R⁴ represents a hydrogen atom or a protective group.
[28] The compound according to any one of [1] to [27], which is used to select a peptide compound having binding selectivity for a target substance from a peptide compound library.
[29] A conjugate, comprising the compound according to any one of [1] to [28] and a target substance linked to the compound.
[30] The conjugate according to [29], wherein the target substance is a protein.
[31] A method for producing a compound having a photocleavable moiety, and a target substance binding site or anchor binding site,
   comprising a step of linking a precursor compound A comprising a photocleavable moiety, to a precursor compound B comprising a target substance binding site or an anchor binding site.
[32] The method according to [31], wherein the linkage of the photocleavable moiety to the target substance binding site or the anchor binding site is made via a spacer arm.
[33] A method for selecting a substance of interest capable of binding to a target substance, comprising:
   a contacting step of bringing a library of object substances into contact with a conjugate comprising a compound having a photocleavable moiety and the target substance linked to the compound, and
   an elution step of irradiating the conjugate with light to obtain the substance of interest.
[34] The method according to [33], wherein the object substance comprises a nucleic acid.
[35] The method according to [33] or [34], wherein the object substance is a peptide compound linked to a nucleic acid.
[36] The method according to any one of [33] to [35], wherein the library is a library tagged by a nucleic acid.
[37] The method according to any one of [33] to [36], wherein the compound and the target substance are linked by an enzymatic reaction.
[38] The method according to [37], wherein the enzyme used in the enzymatic reaction is a sortase.
[39] The method according to any one of [33] to [38], wherein the wavelength of the light is 300 nm or more and 500 nm or less.
[40] The method according to any one of [33] to [39], wherein the illuminance of the light is 10 mW/cm² or more and 600 mW/cm² or less.
[41] The method according to any one of [33] to [40], wherein the irradiation time of the light is 0.5 minutes or more and 60 minutes or less.
[42] The method according to any one of [33] to [41], wherein heat elution and enzymatic reaction are not used in the elution step.
[43] The method according to any one of [33] to [42], wherein the elution step is performed after the contacting step.
[44] The method according to any one of [33] to [43], wherein the contacting step and the elution step are repeated two or more times.
[45] The method according to any one of [33] to [44], wherein the compound is the compound according to any one of [1] to [28].
[46] The method according to any one of [33] to [44], wherein the conjugate is the conjugate according to [29] or [30].

### [Advantageous Effect of Invention]

According to the present invention, it is possible to provide a compound which can be utilized for a novel panning that uses light for elution.

### [Brief Description of Drawings]

[Figure 1] Figure 1 is a photograph showing the results of Western blotting in Evaluation Example 1-1.
[Figure 2] Figure 2 is a photograph showing the results of capillary electrophoresis in Evaluation Example 1-2.
[Figure 3] Figure 3 is a photograph showing the results of capillary electrophoresis in Evaluation Example 1-3.
[Figure 4] Figure 4 is a photograph showing the results of capillary electrophoresis in Evaluation Example 1-4.

### [Description of Embodiments]

Hereinafter, the mode for carrying out the present invention will be described in detail. However, the present invention is not limited by embodiments given below.

### [Terms in the Present Specification]

As used herein, the term "one or more" means a number of 1 or 2 or larger. When the term "one or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, and/or larger numbers.

As used herein, the term "to" that indicates a range includes values of both ends thereof. For example, "A to B" means the range of A or more and B or less.

As used herein, the term "approximately", when used in combination with a numeric value, means the value range of +10% and -10% of the numeric value.

As used herein, the term "and/or" is meant to include every combination of the terms "and" and "or" appropriately combined. Specifically, for example, the term "A, B, and/or C" includes the following seven variations: (i) A, (ii) B, (iii) C, (iv) A and B, (v) A and C, (vi) B and C, and (vii) A, B, and C.

As used herein, the "C1-6 alkyl group" refers to an alkyl group having 1 to 6 carbon atoms. The C1-6 alkyl group may be linear or branched. Examples of the C1-6 alkyl group include a methyl group, an ethyl group, an n-propyl group, an isopropyl group, an n-butyl group, a sec-butyl group, a tert-butyl group, a 1-methylpropyl group, an n-pentyl group, a 1-methylbutyl group, a 2-methylbutyl group, a 3-methylbutyl group, a 1,1-dimethylpropyl group, a 2,2-dimethylpropyl group, a 1,2-dimethylpropyl group, a 1-ethylpropyl group, an n-hexyl group, a 1-methylpentyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethylbutyl group and a 2-ethylbutyl group.

As used herein, the "C1-10 alkoxy group" is an alkyl group having 1 to 10 carbon atoms bonded to an oxygen atom. The C1-10 alkoxy group may be, for example, a C1-6 alkoxy group that is a group where the above C1-6 alkyl group is bonded to an oxygen atom. Examples of the C1-6 alkoxy group include a methoxy group, an ethoxy group, an n-propoxy group, an isopropoxy group, an n-butoxy group, a sec-butoxy group, a tert-butoxy group, an n-pentoxy group, and an n-hexoxy group.

As used herein, the "C1-10 alkylene group" refers to a divalent group induced by the removal of one arbitrary hydrogen atom from an alkyl group having 1 to 10 carbon atoms. The C1-10 alkylene group may be, for example, a C1-6 alkylene group that is a divalent group induced by the removal of one arbitrary hydrogen atom from the above C1 - 6 alkyl group. Examples of the C1-6 alkylene group include a methylene group, an ethylene group, a 1,2-propylene group, an n-propylene group, a 1,2-butylene group, a 1,3-butylene group, an n-butylene group, a 2,3-butylene group, an n-pentylene group, and an n-hexylene group.

As used herein, the term "amino acid" includes a natural amino acid and a non-natural amino acid (sometimes also referred to as an amino acid derivative). Also, as used herein, the term "amino acid residue" includes a natural amino acid residue and a non-natural amino acid (amino acid derivative) residue.

Natural amino acids refer to glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro).

Non-natural amino acids (amino acid derivatives) are not particularly limited, and examples thereof include a β-amino acid, a D-type amino acid, an N-substituted amino acid (excluding Pro), an α,α-disubstituted amino acid, an amino acid having a side chain different from that of natural amino acids, and a hydroxycarboxylic acid.

As the amino acids herein, amino acids having any conformation are acceptable. The selection of a side chain of an amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. Each of the side chains may have a substituent. The substituent is also not limited, and one or two or more substituents may be freely selected independently from any substituents including, for example, a halogen atom, an O atom, a S atom, a N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group which may be substituted, oxo, aminocarbonyl, and a halogen atom. The amino acid according to one embodiment may be a compound having a carboxy group and an amino group in the same molecule, and even in this case, imino acids such as proline and hydroxyproline are also included in the amino acid.

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C=O-R), carboxy (-CO₂H), oxycarbonyl (-C=O-OR), carbonyloxy (-O-C=O-R), thiocarbonyl (-C=O-SR), a carbonylthio group (-S-C=O-R), aminocarbonyl (-C=O-NHR), carbonylamino (-NH-C=O-R), oxycarbonylamino (-NH-C=O-OR), sulfonylamino (-NH-SO₂-R), aminosulfonyl (-SO₂-NHR), sulfamoylamino (-NH-SO₂-NHR), thiocarboxy (-C(=O)-SH), and carboxylcarbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of the carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include groups in which the H atom bonded to the N atom in -C=O-NHR is further replaced with alkyl, cycloalkyl, alkenyl, alkynyl, aryl, heteroaryl, or aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Examples thereof additionally include groups in which the H atom bonded to the N atom in -NH-C=O-R is further replaced with alkyl, cycloalkyl, alkenyl, alkynyl, aryl, heteroaryl, or aralkyl.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxy carbonylamino, and aralkyloxycarbonylamino. Examples thereof additionally include groups in which the H atom bonded to the N atom in -NH-C=O-OR is further replaced with alkyl, cycloalkyl, alkenyl, alkynyl, aryl, heteroaryl, or aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Examples thereof additionally include groups in which the H atom bonded to the N atom in -NH-SO₂-R is further replaced with alkyl, cycloalkyl, alkenyl, alkynyl, aryl, heteroaryl, or aralkyl.

Examples of aminosulfonyl (-SO₂-NHR) include alkylaminosulfonyl, cycloalkylaminosulfonyl, alkenylaminosulfonyl, alkynylaminosulfonyl, aryl aminosulfonyl, heteroarylaminosulfonyl, and aralkylaminosulfonyl. Examples thereof additionally include groups in which the H atom bonded to the N atom in -SO₂-NHR is further replaced with alkyl, cycloalkyl, alkenyl, alkynyl, aryl, heteroaryl, or aralkyl.

Examples of sulfamoylamino (-NH-SO₂-NHR) include alkylsulfamoylamino, cycloalkylsulfamoylamino, alkenylsulfamoylamino, alkynyl sulfamoyl amino, arylsulfamoylamino, heteroarylsulfamoylamino, and aralkylsulfamoylamino. Further, the two H atoms bonded to the N atom in -NH-SO₂-NHR may be substituted with substituents independently selected from the group consisting of alkyl, cycloalkyl, alkenyl, alkynyl, aryl, heteroaryl, and aralkyl, or the two substituents may form a ring.

Examples of S-atom-derived substituents include thiol (-SH), thio (-S-R), sulfinyl (-S=O-R), sulfonyl (-S(O)₂-R), sulfo (-SO₃H), and pentafluorosulfanyl (-SF₅).

Examples of thio (-S-R) include alkylthio, cycloalkylthio, alkenylthio, alkynylthio, arylthio, heteroarylthio, and aralkylthio.

Examples of sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of N-atom-derived substituents include azido (-N₃, also referred to as "azide group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR"')-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include an amino group having any two substituents each independently selected from alkyl, cycloalkyl, alkenyl, alkynyl, aryl, heteroaryl, aralkyl, and the like, such as alkyl(aralkyl)amino, and the two substituents may form a ring.

Examples of substituted amidino (-C(=NR)-NR'R") include groups in which the three substituents R, R', and R" on the N atom are each independently selected from alkyl, cycloalkyl, alkenyl, alkynyl, aryl, heteroaryl, and aralkyl. Examples of substituted amidino include alkyl(aralkyl)(aryl)amidino.

Examples of substituted guanidino (-NR-C(=NR"')-NR'R") include a group in which R, R', R", and R‴ are each independently selected from alkyl, cycloalkyl, alkenyl, alkynyl, aryl, heteroaryl, and aralkyl, and a group in which they form a ring.

Examples of aminocarbonylamino (-NR-CO-NR'R") include a group in which R, R', and R" are each independently selected from a hydrogen atom, alkyl, cycloalkyl, alkenyl, alkynyl, aryl, heteroaryl, and aralkyl, and a group in which they form a ring.

Examples of B-atom-derived substituents include boryl (-BR(R')), and dioxyboryl (-B(OR)(OR')). The two substituents R and R' may be groups each independently selected from alkyl, cycloalkyl, alkenyl, alkynyl, aryl, heteroaryl, aralkyl, or the like, or may form a ring. Examples of B-atom-derived substituents specifically include a cyclic boryl group, and more specifically include a pinacolate boryl group, a neopentanediolate boryl group, and a catecholate boryl group.

The amino group of the main chain of the amino acid may be unsubstituted (-NH₂) or substituted (i.e., -NHR, wherein R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position α may form a ring, like proline).

As used herein, an amino acid in which the amino group of the main chain is substituted is referred to as an "N-substituted amino acid". Examples of the "N-substituted amino acid" as used herein include preferably, but are not limited to, N-alkylamino acid, N-C₁-C₆ alkylamino acid, N-C₁-C₄ alkylamino acid, N-methylamino acid, N-C₇-C₁₄ aralkyl amino acid, N-benzylamino acid, and N-phenethylamino acid.

Specific examples of the substituent on the nitrogen atom of the N-substituted amino acid (R of -NHR described above) as used herein include an alkyl group (preferably a C₁-C₆ alkyl group, more preferably a C₁-C₄ alkyl group, further preferably a methyl group), a C₇-C₁₄ aralkyl group, a benzyl group, and a phenethyl group. As the substituent on the nitrogen atom of the N-substituted amino acid, a methyl group is particularly preferred (i.e., N-methylamino acid is particularly preferred as the N-substituted amino acid).

The "amino acid" as used herein includes all corresponding isotopes to each. The isotope of "amino acid" is one in which at least one atom is substituted with an atom having the same atomic number (the same number of protons) and a different mass number (different sum of numbers of protons and neutrons). Examples of the isotope included in the "amino acid" as used herein include a hydrogen atom, a carbon atom, a nitrogen atom, an oxygen atom, a phosphorus atom, a sulfur atom, a fluorine atom, and a chlorine atom, and they include ²H, ³H; ¹³C, ¹⁴C; ¹⁵N; ¹⁷O, ¹⁸O; ³²P; ³⁵S; ¹⁸F; ³⁶Cl; and the like respectively.

### [Compound]

The compound according to the present embodiment (hereinafter also referred to as "the present compound") has (i) a photocleavable moiety, and (ii) at least one selected from the group consisting of a target substance binding site and an anchor binding site. The present compound may be a compound having (i) a photocleavable moiety and (ii-1) a target substance binding site, a compound having (i) a photocleavable moiety and (ii-2) an anchor binding site, or a compound having (i) a photocleavable moiety, (ii-1) a target substance binding site, and (ii-2) an anchor binding site. The photocleavable moiety, target substance binding site, and anchor binding site may be bound to each other via a spacer arm or without a spacer arm.

There may be a plurality of target substance binding sites in the present compound and they may be of a plurality of types. For example, each side of the present compound can also be designed as a different type of target substance binding site, each binding to a different target substance. In such case, the main chain structure can be branched and the third end can be used as an anchor binding site.

### <Photocleavable Moiety>

The photocleavable moiety is a moiety that is cleaved by absorbing light. The absorption wavelength at which the photocleavable moiety undergoes cleavage may be 300 nm or more, 320 nm or more, 340 nm or more, or 350 nm or more, and may be 500 nm or less, 450 nm or less, 400 nm or less, 380 nm or less, or 370 nm or less. The absorption wavelength at which the photocleavable moiety undergoes cleavage may be 300 nm or more and 500 nm or less, or 350 nm or more and 370 nm or less. The wavelength of the irradiated light may be a wavelength in the UV-A region from the viewpoint of better handling.

A wavelength in the UV-A region refers to light with a wavelength of 320 to 400 nm.

The photocleavable moiety may have a nitroveratryloxycarbonyl residue or a coumarin residue.

Having a nitroveratryloxycarbonyl residue means having a structure represented by the following formula (A-1) or a structure in which some atoms of the structure are replaced by other atoms.

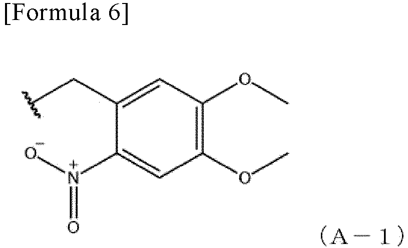

Having a coumarin residue means having a structure represented by the following formula (A-2) or a structure in which some atoms of the structure are replaced by other atoms. 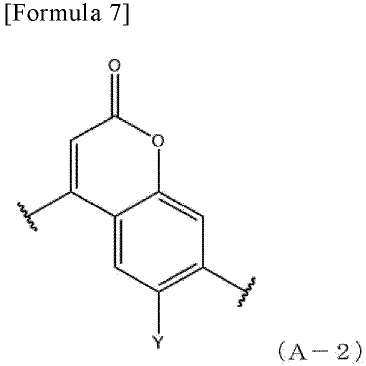 (In formula (A-2), Y represents a hydrogen atom or a halogen atom.)

When the photocleavable moiety has a nitroveratryloxycarbonyl residue or a coumarin residue, the absorption wavelength at which the photocleavable moiety undergoes cleavage is a suitable wavelength (e.g., a wavelength in the UV-A region).

The photocleavable moiety may have a structure represented by the following formula (1), which is a nitroveratryloxycarbonyl residue, because it has a suitable absorption wavelength (e.g., a wavelength in the UV-A region) for causing cleavage. In formula (1), * represents a bond.

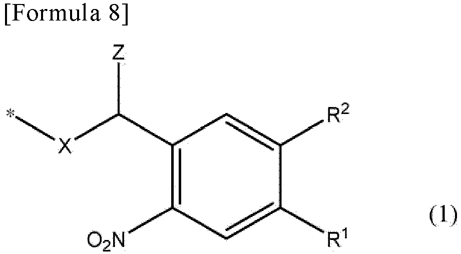

X represents O or NH. X may be bound to the spacer arm or target substance binding site described below.

Z represents a hydrogen atom or a C1-6 alkyl group. Z may be a C1-6 alkyl group, and may be a methyl group.

R¹ represents -O-L¹¹-L¹²-*, a C1-10 alkoxy group, or a hydrogen atom, R² represents -O-L¹¹-L¹²-* or a C1-10 alkoxy group, L¹¹ represents a C1-10 alkylene group, and L¹² represents a divalent group selected from the group consisting of a single bond, a C1-10 alkylene group, an amide bond, an ether bond and combinations thereof. L¹² may be bound to the spacer arm or anchor binding site described below.

Any one of R¹ and R² is -O-L¹¹-L¹²-*. If R² is -O-L¹¹-L¹²-*, R¹ is a C1-10 alkoxy group or a hydrogen atom. If R¹ is -O-L¹¹-L¹²-*, R² may be a C1-10 alkoxy group, and may be a methoxy group.

The -O-L¹¹-L¹²-* as R¹ or R² may be a group where L¹¹ is a C1-10 alkylene group and L¹² is an amide bond, and may be, for example, -O-CH₂-CH₂-CH₂-CONH-*.

The photodegradable moiety having a structure represented by formula (1) may have a structure represented by the following formula (1a). In formula (1a), * represents a bond.

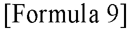

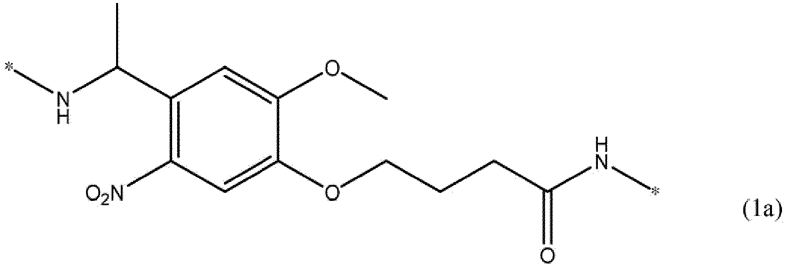

The photocleavable moiety may have a structure represented by the following formula (2), which is a coumarin residue, because it has a suitable absorption wavelength (e.g., a wavelength in the UV-A region) for causing cleavage. In formula (2), * represents a bond.

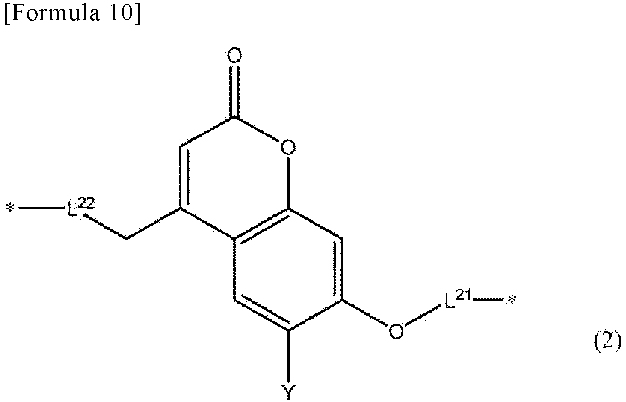

In the formula, Y represents a hydrogen atom or a halogen atom. The halogen atom represented by Y may be a bromine atom (-Br), an iodine atom (-I), a chlorine atom (-Cl), or a fluorine atom (-F), and is preferably a bromine atom.

L²¹ and L²² each independently represent a divalent group selected from the group consisting of a single bond, a C1-10 alkylene group, an amide bond, an ether bond, and combinations thereof.

L²¹ may be a group consisting of a C1-10 alkylene group and an amide bond, and, for example, may be -CH₂-CH₂-CH₂-CONH-. L²¹ may be bound to the spacer arm or anchor binding site described below.

L²² may be -O-CONH-. L²² may be bound to the spacer arm or target substance binding site described below.

The photodegradable moiety having a structure represented by formula (2) may have a structure represented by the following formula (2a). In formula (2a), * represents a bond.

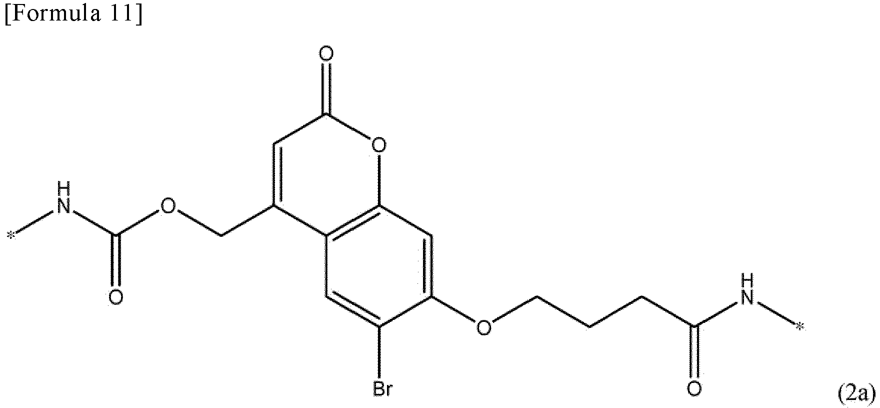

The present compound may have one or more photocleavable moieties. If the present compound has a plurality of photocleavable moieties, it is possible to more efficiently enrich the compound that binds to the target when used for panning. The number of photocleavable moieties in the present compound may be 1 or more, 2 or more, 3 or more, or 4 or more, and may be 10 or less, 9 or less, 8 or less, 7 or less, 6 or less, 5 or less, or 4 or less. The number of photocleavable moieties in the present compound may be, for example, 2 or more and 5 or less.

If the compound has a plurality of photocleavable moieties, the photocleavable moieties may all have the same structure, or may partially differ. In the present compound, the plurality of photocleavable moieties may all have the structure represented by formula (1) or formula (1a) above, or may all have the structure represented by formula (2) or formula (2a) above.

### <Target Substance Binding Site>

The target substance binding site refers to a site that can be linked to a target substance by a chemical bond or the like. The target substance binding site includes, for example, a structure recognized by an enzyme (such as an amino acid sequence) or the structure to which a protective group is added. Having a target substance binding site allows the present compound to be linked to a target substance, such as a protein or peptide.

The target substance binding site may be an enzyme recognition site recognized by an enzyme, and may be a site recognized by at least one selected from the group consisting of ligases and proteases. The ligase may be a sortase such as Sortase A, Sortase B, or Sortase C, Butelase-1, which is a type of peptide asparaginyl ligase (plant-derived), OaAEP1, which is a type of asparaginyl endopeptidase (plant-derived), Trypsiligase, or a mutant thereof.

Taking as an example the present compound containing the N-terminal recognition sequence GGG (Gly-Gly-Gly) of Sortase A as the target substance binding site, the reaction for binding the present compound to a target substance will be described. Here, Sortase A is an enzyme that cleaves the bond between T and G in the C-terminal recognition sequence LPXTG (Leu-Pro-X-Thr-Gly. X: any amino acid residue), and catalyzes the reaction of linking a protein or peptide having a G(n) sequence at the N-terminus to the C-terminal side of the cleaved T by a peptide bond (n: an integer of 2 or larger). The N-terminal G can also be replaced by an A (Ala).

As shown in the following exemplary scheme, first a starting material SM (protein/peptide-LPXTGG), which is a protein or peptide containing the C-terminal recognition sequence LPXTG of Sortase A, is prepared, and reacted with Sortase A (SrtA-SH) to form an intermediate IM. The intermediate IM then reacts with the present compound (GGG-Z) containing the N-terminal recognition sequence GGG of Sortase A as the target substance binding site, thereby linking the target substance to the present compound and forming the substance of interest TM (protein/peptide-LPXTGGG-Z).

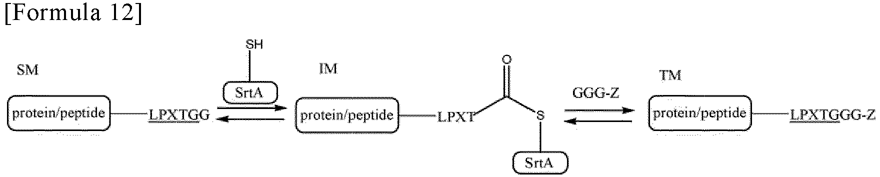

In the above scheme, the targets to which the C-terminal recognition sequence LPXTG and the N-terminal recognition sequence GGG of Sortase A bind may be interchanged. That is, it is also possible to obtain Z-LPXTGGG-protein/peptide as the target substance TM by preparing the present compound (Z-LPXTG) containing LPXTG as the starting material SM, reacting it with Sortase A (SrtA-SH) to form Z-LPXT-C(=O)S-SrtA, which is the intermediate IM, and then reacting the intermediate IM with a protein or peptide containing GGG (GGG-protein/peptide).

The same can be said for all target substance binding sites hereinafter.

Sortase B is an enzyme that recognizes the NPQTN (Asn-Pro-Gln-Thr-Asn) motif, while Sortase C is an enzyme that recognizes the QVPTGV (Gln-Val-Pro-Thr-Gly-Val) motif.

When Butelase-1 is used as the enzyme, the C-terminal recognition sequence is D/NHV (Asp/Asn-His-Val), the N-terminal recognition sequence is X (X is any amino acid residue), and the generated sequence post-reaction is D/NX (Asp/Asn-X).

When OaAEP1 is used as the enzyme, the C-terminal recognition sequence is NXL (Asn-X-Leu), the N-terminal recognition sequence is GY (Gly-Tyr), and the generated sequence post-reaction is NGY (Asn-Gly-Tyr). OaAEP1 can be made into a C247A mutant to improve ligase activity.

When Trypsiligase is used as the enzyme, the C-terminal recognition sequence is YRH (Tyr-Arg-His), the N-terminal recognition sequence is RH (Arg-His), and the generated sequence post-reaction is YRH (Tyr-Arg-His).

The structure of the target substance binding site is appropriately selected according to the type of enzyme. The target substance binding site may contain one or more amino acid residues and may contain two or more amino acid residues. The number of amino acid residues of the target substance binding site may be, for example, 5 or less, 4 or less, or 3 or less. When the target substance binding site contains two or more amino acid residues, it may contain only the same amino acid residues, or different amino acid residues.

The target substance binding site may contain, for example, the N-terminal recognition sequence or C-terminal recognition sequence of the enzymes described above. The target substance binding site may contain, for example, one or more glycine residues, two or more glycine residues, or three glycine residues.

The amino acid residues in the target substance binding site may be protected with a protective group. For example, the main chain amino group and/or carboxy group of the amino acid residue may be protected, and the side chain functional group may be protected. The protective group protecting these is not limited, but the protecting group of the main chain amino group may be, for example, at least one selected from the group consisting of a 9-fluorenylmethyloxycarbonyl (Fmoc) group, a tert-butoxycarbonyl (Boc) group, a benzyloxycarbonyl (Cbz) group, a 2-nitrobenzenesulfonyl (Ns) group and an allyloxycarbonyl (Alloc) group. The protective group of the carboxy group may be at least one selected from the group consisting of methyl esters, ethyl esters, tert-butyl esters, benzyl esters (substituents may be present on the aromatic ring moiety), and trityl esters, and may be at least one selected from the group consisting of 2,4-bis(dodecyloxy)benzyl) and 9-phenylfluorenyl, which are protective groups used in methods in which the solubility in various solvents is adjusted to efficiently produce peptide compounds by precipitation (Okada, Y. et al.: J. Org. Chem., 78, 320 (2013); Takahashi, D. and Yamamoto, T.: Tetrahedron Lett., 53, 1936 (2012); and Takahashi, D. et al.: Angew. Chem. Int. Ed., 56, 7803 (2017)). As the protective group of the side chain functional group, an appropriate protective group for each side chain functional group can be arbitrarily selected.

The target substance binding site may have a structure represented by the formula (B): R⁴-(X)ₙ-*. In formula (B), X represents any amino acid residue, R⁴ represents a hydrogen atom or a protective group, and n represents an integer of 1 or larger. * represents a bond. * may be a binding site for the above photocleavable moiety or the spacer arm described below.

The amino acid residue represented by X and the protective group represented by R⁴ may be as illustrated above. n may be 2 to 5 or 2 to 4. Formula (B) may specifically be R⁴-GGG-. Here, G represents Gly.

### <Anchor Binding Site>

The anchor binding site is a site that allows binding to a carrier used in panning or the like. The anchor binding site contains a tag having high affinity for a particular substance. The binding of a carrier retaining a substance with high affinity for the tag, to the tag in the anchor binding site allows the target substance to be retained on the carrier. Here, the carrier is preferably a solid phase.

Examples of combinations of tags and substances with high affinity for the tags include the combination of a biotin tag with a biotin-binding protein, the combination of a PA tag with an anti-PA tag antibody, the combination of a FLAG tag with an anti-FLAG tag, and the combination of a His tag with Ni-NTA (nitrilotriacetic acid).

The anchor binding site may be a biotin tag. In this case, examples of the biotin-binding protein include avidin, streptavidin, neutravidin, and anti-biotin antibodies. The biotin is preferably D-biotin.

The anchor binding site may have, for example, a structure represented by the following formula (C):

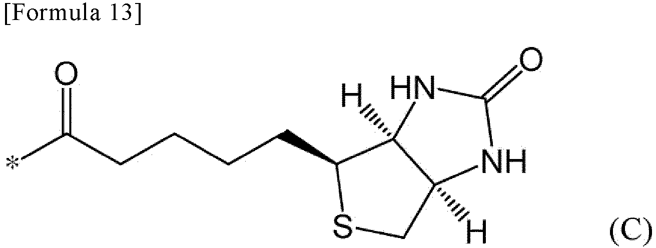

### <Spacer Arm>

The spacer arm is a site that binds photocleavable moieties to each other, a photocleavable moiety and a target substance binding site, or a photocleavable moiety and an anchor binding site to each other. If there is a plurality of photocleavable moieties, some or all of the plurality of photocleavable moieties may be bound via the spacer arm. The spacer arm allows to more easily control the lipid solubility of the present compound. By controlling the lipid solubility of the present compound, it is thought that the decrease in reaction efficiency caused by precipitation from the reaction solution, adsorption to the reaction vessel, or the like when binding to the target substance is more easily suppressed, and aggregation of the target substance is less likely to occur after the reaction with the target substance and purification, which in turn allows to more easily control the decrease in recovery ratio during the enrichment of the target substance when used for panning.

The spacer arm may contain an oxyethylene group (-CH₂-CH₂-O-), an amino acid residue, or a substituted or unsubstituted alkylene group. The substituted alkylene group may be an alkylene group in which one or more hydrogen atoms are optionally substituted with a substituent. The alkylene group in the substituted or unsubstituted alkylene group may be the above C1-10 alkylene group.

The spacer arm may contain a C1-10 alkylene group, it may contain an ether group (-O-), or it may contain a C1-10 alkylene group and an ether group. The spacer arm may contain -NH-.

The amino acid residue in the spacer arm may be, for example, a glycine residue, a serine residue, a threonine residue, or a lysine residue. If the spacer arm contains an amino acid residue, it may be bound to the anchor binding site or photocleavable moiety via a side chain functional group of the amino acid residue (e.g., the ε-amino group of a lysine residue).

The spacer arm may contain at least one selected from the group consisting of a polyethylene glycol residue, a polyamine residue, a glycine residue, a serine residue and a threonine residue.

The spacer arm may have, for example, a structure represented by the following formulas (D1), (D2), or (D3):

*-CH₂-CH₂-O-CH₂-CH₂-O-CH₂-C(=O)-* (D1)

*-NH-CH₂-CH₂-O-CH₂-CH₂-* (D2)

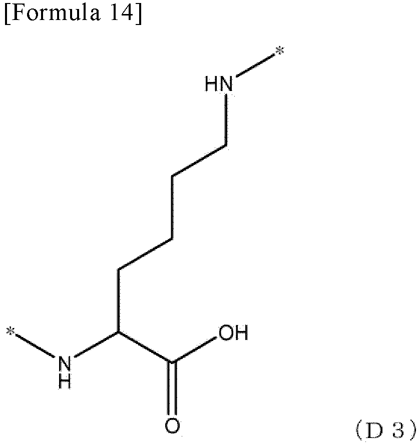

The present compound may have a plurality of structural units consisting of photocleavable moieties and spacer arms. In this case, it is possible to more efficiently enrich the compound that binds to the target when used for panning. The number of structural units consisting of photocleavable moieties and spacer arms per molecule of the present compound may be 1 or more, 2 or more, 3 or more, or 4 or more, and may be 10 or less, 9 or less, 8 or less, 7 or less, 6 or less, 5 or less, or 4 or less. If the present compound has a plurality of structural units consisting of spacer arms, it may contain structural units consisting of two or more spacer arms. The present compound has preferably one structural unit consisting of a spacer arm.

The structural unit consisting of a photocleavable moiety and a spacer arm may be, for example, of the following formula (E1) or (E2).

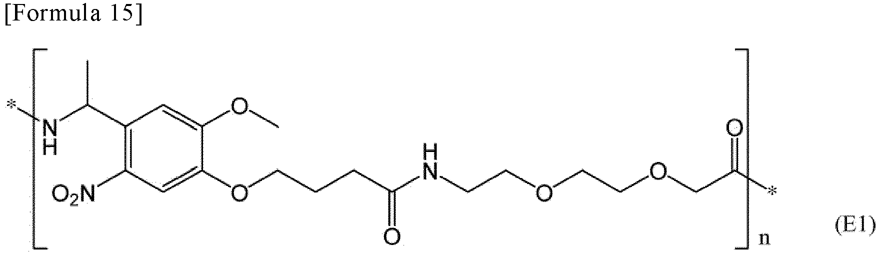

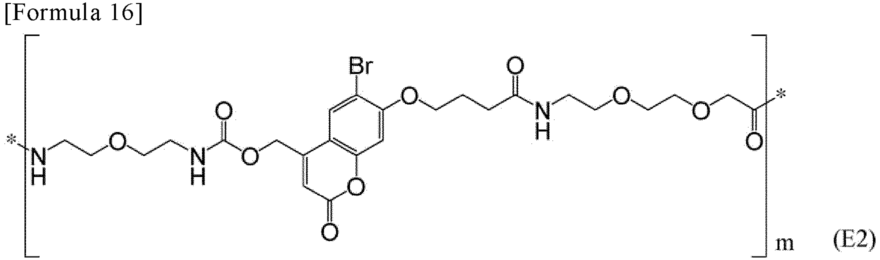

In the formula, n represents an integer of 1 or larger and 5 or smaller, and m represents an integer of 1 or larger and 5 or smaller.

### <Compound>

The present compound may be a compound represented by the following formula (3) or (4), which contains a target substance binding site, a photocleavable moiety, an anchor binding site and a spacer arm.

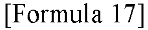

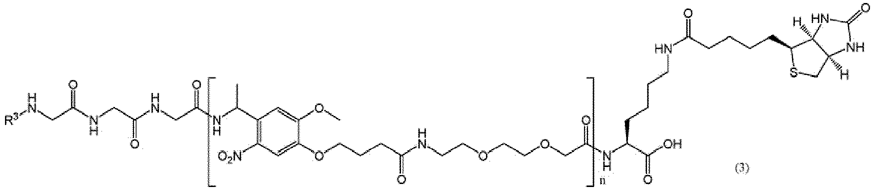

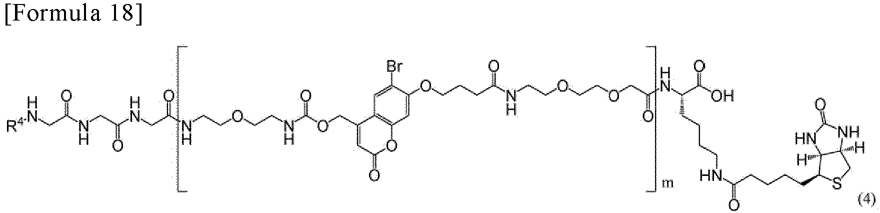

In formula (3), m represents an integer of 1 or larger and 5 or smaller, and R³ represents a hydrogen atom or a protective group. In formula (4), n represents an integer of 1 or larger and 5 or smaller, and R⁴ represents a hydrogen atom or a protective group. The protective groups in R³ and R⁴ may be the protective groups of the amino acid residues listed above.

### <Use of Compound>

The present compound can be utilized, for example, for panning that uses light when eluting. For example, other than using light for elution, panning using the present compound can be performed by usual methods. The present compound can be used to form an immobilized target substance. Using the present compound in the formation of an immobilized target substance allows to elute the compound bound to the target substance (compound of interest) by light.

When the compound of interest is eluted by heat, non-specific binding molecules physically adsorbed on the carrier (beads, etc.) may be eluted together with the compound of interest, making it difficult to selectively obtain the compound of interest. The compound according to the present embodiment allows elution at low temperatures, unlike elution by heat, since the compound of interest is eluted by light. Therefore, using the present compound for panning and eluting the compound of interest by light allows to limit the amount of non-specific binding molecules recovered and to more selectively obtain the substance of interest.

When the compound of interest is eluted using an enzyme such as TEV (Tobacco Etch Virus) protease, in the step of eluting the compound of interest, for example, the elution efficiency may vary depending on the target substance, and the amount of non-specific binding molecules recovered may increase due to the TEV protease, buffer exchange, heating, and the like. The present compound allows to elute the substance of interest at a low temperature and in a short time, regardless of the target substance, since the compound of interest is eluted by light. In addition, since TEV protease may inhibit PCR, the efficiency of DNA amplification by PCR may be low, especially in the early stages of panning (the stages with a small number of rounds of panning), when the amount of compound of interest recovered is small. Photoelution using the present compound does not inhibit PCR and is less likely to reduce the efficiency of amplification by PCR.

As described above, photoelution using the present compound allows to reduce the elution of non-specific binding molecules. Therefore, it can be expected to improve the recovery ratio of the compound of interest (hit compound) and to accelerate the panning step (e.g., to be able to determine whether it is a hit or not at a stage where the number of panning rounds is small).

Since the present compound can be utilized for panning using light when eluting, it can be used to select a peptide compound from a peptide compound library that has binding selectivity for the target substance.

### <Method for Producing Compound>

The compound according to the present embodiment can be produced, for example, by a method including a step of linking a precursor compound A containing a photocleavable moiety, to a precursor compound B containing a target substance binding site or an anchor binding site.

The linkage of the photocleavable moiety to the target substance binding site or the anchor binding site may be made via a spacer arm.

The precursor compound A may contain a spacer arm. Some of the functional groups in the precursor compound A or precursor B may be protected with a protective group if necessary. The protective group may be, for example, the protective groups of the amino acid residues listed above.

The method may be performed by a liquid-phase synthesis method or by a solid-phase synthesis method. As a solid-phase synthesis method, general solid-phase synthesis methods can be used.

An example of the method for producing a compound containing a target substance binding site, a photocleavable moiety, an anchor binding site and a spacer arm will be described below.

The method may include, for example, a step 1 of linking a precursor compound a1 containing a photocleavable moiety to a precursor compound b containing a spacer arm to obtain an intermediate X1, a step 2 of linking the intermediate X1 to a precursor compound c containing an anchor binding site to obtain an intermediate X2, and a step 3 of linking the intermediate X2 to a precursor compound d containing a target substance binding site to obtain the present compound of interest.

After step 2 and before step 3 may be included the linking of a precursor compound a2 containing a photocleavable moiety to the intermediate X2. This allows to obtain an intermediate X2 having a plurality of photocleavable moieties. After step 2 and before step 3, the linking of the precursor compound a2 containing a photocleavable moiety to the intermediate X2 may be performed a plurality of times. The precursor compound a2 may contain a spacer arm in addition to a photocleavable moieity.

The precursor compounds and intermediates used in each step may be protected by a protective group. The method may include a step of deprotecting the protective group between each step, if necessary.

More specifically, it is possible to produce a compound containing a target substance binding site, a photocleavable moiety, an anchor binding site, and a spacer arm according to the reaction scheme and reaction conditions described in the Examples below.

### [Conjugate]

The conjugate according to the present embodiment contains the present compound and a target substance linked to the compound. That is, the conjugate contains a target substance and a photocleavable moiety. The conjugate may further contain an anchor binding site.

The target substance is, for example, a protein, a peptide, or a nucleic acid. Examples of target substances include KRAS, NusA, sfGFP, and GST.

When using a compound containing a target substance binding site, the conjugate according to the present embodiment can be obtained by reacting the present compound with a target substance in a reaction mixture containing an enzyme, the present compound, and the target substance, and linking them. More specifically, the conjugate can be obtained under the reaction conditions described in the Examples below.

### [Screening Method]

The method according to the present embodiment is a method for selecting a substance of interest capable of binding to a target substance (a substance of interest having binding selectivity for the target substance). The method can also be called a screening method for substances of interest capable of binding to target substances.

The screening method includes a contacting step of bringing a library of object substances into contact with a conjugate containing the present compound having a photocleavable moiety and a target substance linked to the compound, and an elution step of irradiating the conjugate with light to obtain the substance of interest. In certain aspects, the elution step may be performed after the contacting step. In certain aspects, the contacting step and elution step can be repeated two or more times. The number of repetitions is not limited, but examples thereof include two, three, four, and five. The substance of interest can be enriched by repetition. Here, the library of object substances is a group of object substances composed of a plurality of types of object substances.

The aspects mentioned above can be applied to the photocleavable moiety, the present compound having the photocleavable moiety, the target substance, and the conjugate. The present compound may be linked to the target substance by an enzymatic reaction. The enzyme used in the enzymatic reaction may be one of those listed above and may be a sortase.

Examples of the step of bringing a library of object substances into contact with the conjugate includes a method of adding the conjugate to a liquid containing a group of object substances, and then incubating. The conditions (incubation time, incubation temperature, etc.) under which the library of object substances is brought into contact with the conjugate can be appropriately selected according to the type of target substance, the type of object substance, and the like.

After bringing the library of object substances into contact with the conjugate, a carrier capable of binding to the conjugate may be brought into contact with the conjugate. If the conjugate has an anchor binding site containing a biotin tag, the carrier capable of binding to the conjugate may be, for example, a carrier (such as beads) to which a biotin-binding protein (e.g., streptavidin or an anti-biotin antibody) is bound.

The object substances in the library of object substances that do not bind to the conjugate may be removed after the conjugate and the library of object substances have been brought into contact.

In some aspects, the conjugate is irradiated with light after the contacting step. Irradiation of light allows to cleave the photocleavable moiety and elute the object substance bound to the target substance. This allows to obtain the substance of interest bound to the target substance.

The conditions during light irradiation (wavelength, temperature, time, etc. during irradiation) can be appropriately selected according to the type of photocleavable moiety and the like. For example, the wavelength of the irradiated light may be 300 nm or more, 320 nm or more, 340 nm or more, or 350 nm or more, and may be 500 nm or less, 450 nm or less, 400 nm or less, 380 nm or less, or 370 nm or less. The wavelength of the irradiated light may be 300 nm or more and 500 nm or less, 340 nm or more and 400 nm or less, or 350 nm or more and 370 nm or less. The wavelength of the irradiated light may be a wavelength in the UV-A region from the viewpoint of better handling. The illuminance of the light irradiated may be, for example, 10 mW/cm² or more, 20 mW/cm² or more, or 30 mW/cm² or more, and less than 100 mW/cm², 80 mW/cm² or less, 60 mW/cm² or less, or 50 mW/cm² or less in one embodiment. In other embodiments, the illuminance may be 100 mW/cm² or more, 200 mW/cm² or more, or 300 mW/cm² or more, and 600 mW/cm² or less, 500 mW/cm² or less, or 400 mW/cm² or less. The illuminance of the light irradiated may be, for example, 30 to 50 mW/cm², or 300 to 400 mW/cm². The irradiation time of the light may be, for example, 0.5 minutes or more, 1 minute or more, 1.5 minutes or more, or 2 minutes or more, and less than 5 minutes, 4 minutes or less, or 3 minutes or less in one embodiment. In other embodiments, the irradiation time may be 60 minutes or less, 45 minutes or less, 30 minutes or less, 25 minutes or less, or 20 minutes or less, and 5 minutes or more, or 10 minutes or more. The illuminance and irradiation time of the irradiated light can be appropriately set as desired, but in general, the irradiation time can be shortened when the illuminance is high and lengthened when the illuminance is low.

In some embodiments, heat elution and enzymatic reactions may not be used in the elution step. In the elution step, not using heat elution means not heating to 30°C or more when eluting the object substance bound to the target substance from the carrier.

In the elution step, not using an enzymatic reaction means not using an enzymatic reaction when eluting the object substance bound to the target substance from the carrier, when eluting the object substance bound to the target substance. In the screening method according to some embodiments, no enzyme to elute the object substance is used in the elution step. Examples of enzymes for elution include TEV protease (Tobacco Etch Virus Protease).

In the screening method according to the present embodiment, photoelution is possible, and unlike heat elution, elution at low temperatures is possible, making it easier to selectively obtain the substance of interest and more efficiently enrich the compound of interest. When an enzymatic reaction is used for elution, the subsequent PCR may be inhibited, whereas the screening method according to the present embodiment, which allows elution using light, has not been confirmed to inhibit PCR and has a high DNA amplification efficiency.

The object substance in the screening method according to the present embodiment may include a nucleic acid, and may be, for example, a compound linked to a nucleic acid or a nucleic acid. Examples of object substances include peptide compounds linked to nucleic acids, peptide compounds and antibodies displayed on phage, nucleic acids (RNA or DNA), and low-molecular-weight compounds (e.g., DNA-encoded library). Since photoelution in the present invention allows to more easily selectively obtain the substance of interest than heat elution or enzymatic elution, and that PCR inhibition has not been confirmed, compounds linked to nucleic acids or nucleic acids are preferably exemplified as the object substances in the screening method according to the present embodiment.

A peptide compound linked to a nucleic acid can also be referred to as a peptide compound-nucleic acid complex. A library of peptide compounds linked to nucleic acids can be formed, for example, by the method described in Patent Literature 1.

The library used in the screening method according to the present embodiment is not limited, but examples thereof include libraries tagged (i.e., encoded) by nucleic acids (also referred to as "polynucleotides") (e.g., DNA-encoded libraries; DNA-encoded library/DEL, mRNA display libraries, DNA display libraries; see, e.g., Molecules. 2019 Apr; 24(8): 1629.).

The screening method according to the present embodiment can further include a step of identifying the substance of interest. The substance of interest can be identified by methods well known to those skilled in the art, but, for example, if the object substance is a compound linked to a polynucleotide, the compound linked to the polynucleotide can be identified by determining the nucleotide sequence of the polynucleotide encoding the substance of interest. Specifically, for example, the polynucleotide can be amplified by PCR and its nucleotide sequence analyzed to determine the amino acid sequence or structure of the compound linked to the polynucleotide. If the polynucleotide is mRNA, cDNA synthesized from the mRNA may be used.

The diversity of object substances in the library used in the screening method according to the present embodiment is not limited, but examples thereof include 1 × 10³ or more, 1 × 10⁴ or more, 1 × 10⁵ or more, 1 × 10⁶ or more, 1 × 10⁷ or more, 1 × 10⁸ or more, 1 × 10⁹ or more, 1 × 10¹⁰ or more, 1 × 10¹¹ or more, or 1 × 10¹² or more. These diversities are not limited to measured values, and may be theoretical values. "Diversity" means the number of variations (types) of object substances in the library.

As used herein, the term "peptide compound" is not particularly limited as long as amino acid residues in the peptide compound are linked by an amide bond or an ester bond. The number of amino acid residues in the peptide compound is not particularly limited, but is preferably 5 or more, more preferably 7 or more, further preferably 8 or more, and still more preferably 9 or more. The number of amino acid residues in the peptide compound is also preferably 30 or less, more preferably 25 or less, further preferably 15 or less, and still more preferably 13 or less. The number of amino acid residues of the peptide compound may be, for example, 7 or more and 25 or less, 8 or more and 15 or less, or 9 or more and 13 or less. The peptide compound may have a branched structure.

The peptide compound according to the present embodiments may be a cyclic peptide compound. As used herein, the "cyclic peptide compound" is not particularly limited as long as it is a peptide compound having a cyclic portion. The cyclic portion is preferably formed via a covalent bond such as an amide bond, a carbon-carbon bond formation reaction, an S-S bond, a thioether bond, and a triazole bond. The cyclization may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization through covalent bonds such as amide bonds and carbon-carbon bonds is preferred, and cyclization through an amide bond by a carboxy group of the side chain and an amino group of the main chain is more preferred. The positions of the carboxy group, the amino group, and the like used for cyclization may be on the main chain or the side chain, and are not particularly limited as long as the positions allow the groups to be cyclized.

The cyclic peptide compound may have a linear portion in addition to the cyclic portion. The specific aspects of the number of amino acid residues of the cyclic peptide compound are the same as the specific aspects of the number of amino acid residues of the peptide compound described above. When the cyclic peptide compound has a linear portion, the sum of the numbers of amino acid residues of the cyclic portion and the linear portion preferably falls within the range of the number of amino acid residues of the peptide compound described above. When the cyclic peptide compound has a linear portion, the number of amino acid residues constituting the cyclic portion is preferably 5 or more and 15 or less, 6 or more and 14 or less, or 7 or more and 13 or less, more preferably 7 or more and 12 or less, or 8 or more and 11 or less, further preferably 9 or more and 11 or less, and particularly preferably 10 or 11, and the number of amino acid residues constituting the linear portion is preferably 1 or more and 8 or less, 1 or more and 7 or less, 1 or more and 6 or less, 1 or more and 5 or less, or 1 or more and 4 or less, and more preferably 1 or more and 3 or less.

The molecular weight of the peptide compound according to the present embodiments is not particularly limited, but may be, for example, 500 or more, 550 or more, 600 or more, 650 or more, 700 or more, 750 or more, 800 or more, 850 or more, 900 or more, or 950 or more, and is preferably 1,000 or more, 1,100 or more, 1,200 or more, 1,300 or more, or 1,400 or more. The upper limit of the molecular weight of the peptide compound according to the present embodiments is not particularly limited, but is preferably 5,000 or less, 4,000 or less, 3,000 or less, 2,500 or less, or 2,000 or less.

The screening method according to the present embodiment can be carried out by applying the same procedures, conditions, and the like as in the methods known in the art, except that it includes the above contacting step and above elution step. The screening method according to the present embodiment can be carried out, for example, in the same manner as the method described in Patent Literature 1, except that it includes the above contacting step and above elution step.

### [Examples]

Hereinafter, the present invention will be described more specifically based on Examples. However, the present invention is not limited to the following Examples.

In Examples, the following abbreviations were used.
Acbz: 4-azidobenzyloxycarbonyl group 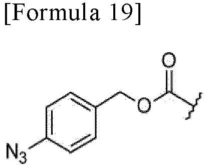
DBU: 1,8-diazabicyclo[5.4.0]-7-undecene
DCM: dichloromethane
DCE: 1,2-dichloroethane
DMF: N,N-dimethylformamide
DIC: N,N'-diisopropylcarbodiimide
DIPEA: N-ethyl-isopropylpropan-2-amine or N,N-diisopropylethylamine
DMSO: dimethyl sulfoxide
DTT: dithiothreitol
EDC·HCI: 1-ethyl-3-(3-dimethylaminopropyl)carbodiimide hydrochloride
FA: formic acid
F-Pnaz: 4-(2-(4-fluorophenyl)acetamido)benzyloxycarbonyl group 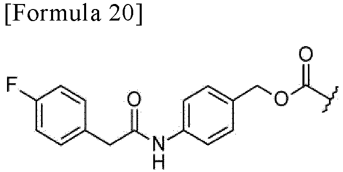
HFIP: 1,1,1,3,3,3-hexafluoroisopropyl alcohol
HOAt: 1-hydroxy-7-azabenzotriazole
HOBt: 1-hydroxybenzotriazole
HOSu: N-hydroxysuccinimide
MeCN: acetonitrile
MeOH: methanol
NMP: N-methyl-2-pyrrolidone
NVOC: 6-nitroveratryloxycarbonyl
PGA: penicillin G acylase
TCEP: tris(2-carboxyethyl)phosphine
TFA: trifluoroacetylacetic acid
TFE: 2,2,2-trifluoroethanol

As the reaction solvents used for solid-phase synthesis, those for peptide synthesis were used (purchased from Watanabe Chemical Industries, Ltd. and FUJIFILM Wako Pure Chemical Corporation). Examples thereof include DCM, DMF, NMP, 2% DBU in DMF, and 20% piperidine in DMF. In addition, in the reactions without addition of water as a solvent, dehydrated solvents, ultra-dehydrated solvents, and anhydrous solvents (purchased from Kanto Chemical Co., Inc., FUJIFILM Wako Pure Chemical Corporation, etc.) were used.

The analysis conditions of LCMS are as described below.

**[Table 1]**

| Analytical Conditions | Device | Column (I.D. x length (mm)) | Mobile Phase | Gradient (A/B) | Flow rate (ml/min) | Column Temperature (°C) | Wavelength |
|---|---|---|---|---|---|---|---|
| SQD | Acquity | Aldrich Ascentis Express C18 2.7µm (2.1 × 50) | A) 0.1% FA, H₂O | 95/5 => 0/100 (1.0 min)=> 0/100 (0.4 min) | 0.9 | 35 | 210-400nm |
| FA05 | UPLC/SQD2 | | B) 0.1% FA CH₃CN | | | | PDA total |
| SMD | Shimadzu | CORTECS C18 2.7µm (2.1 × 50) | A) 0.1% FA, H₂O | 90/10 => 0/100 (1.2 min)=> 0/100 (0.5 min) | 1.0 | 40 | 190-400nm |
| FA10 | LCMS-2020 | | B) 0.1% FA CH₃CN | | | | PDA total |
| | LC-20ADXR | | | | | | |

### Example 1: Chemical synthesis of biotin linker to be bound to target protein

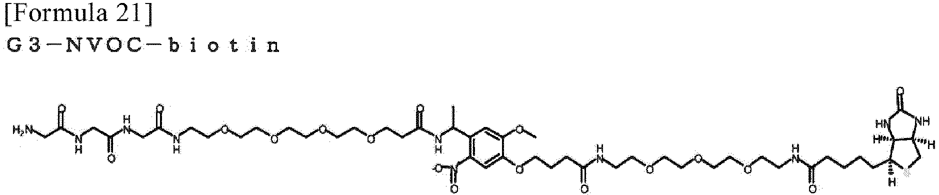

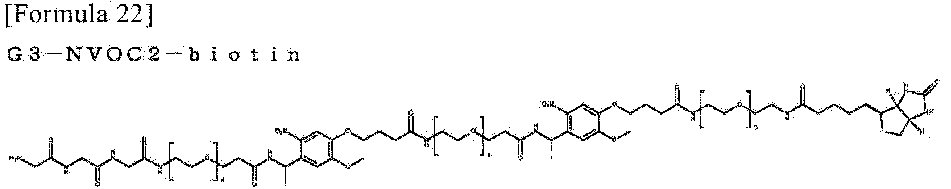

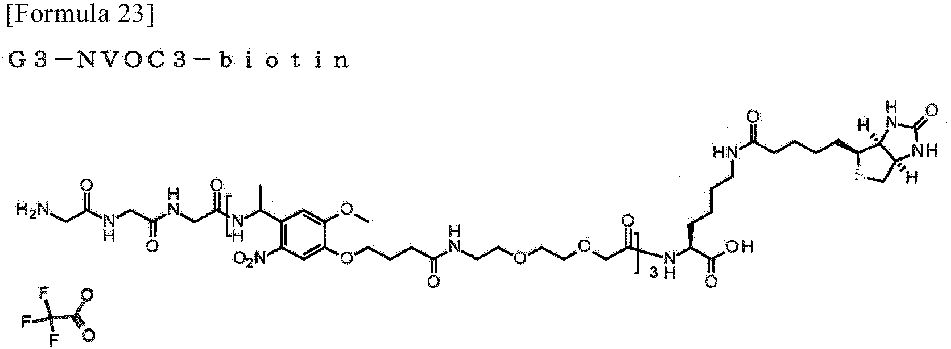

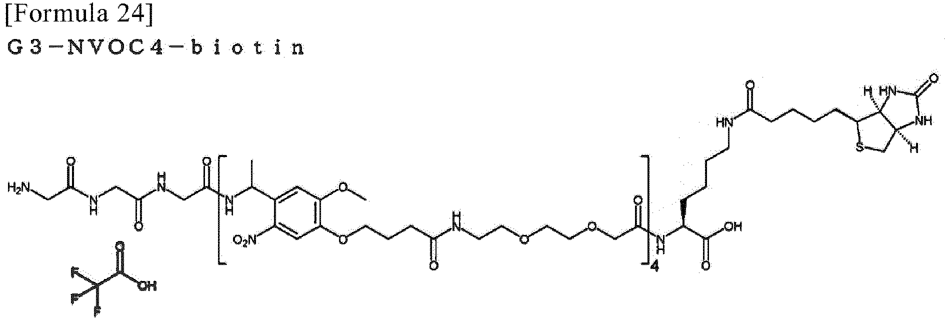

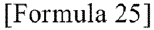

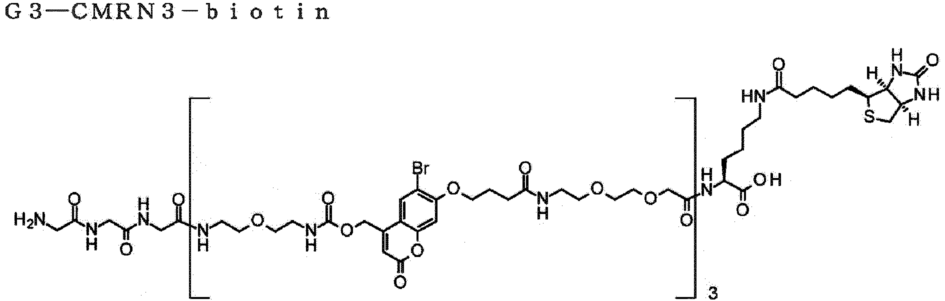

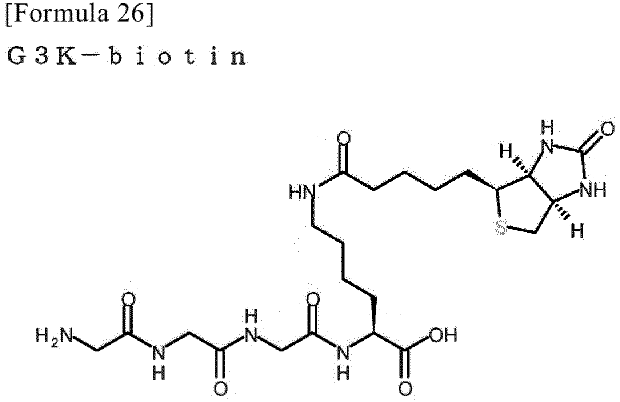

### Example 1-1: Synthesis of G3-NVOC-biotin

### Synthesis of 1-(2-(2-(2-aminoacetamido)acetamido)acetamido)-N-(1-(4-((4,18-dioxo-22-((3aS,4S,6aR)-2-oxohexahydro-1H-thieno[3,4-d]imidazol-4-yl)-8,11,14-trioxa-5,17-diazadocosyl)oxy)-5-methoxy-2-nitrophenyl)ethyl)-3,6,9,12-tetraoxapentadecan-15-amide (G3-NVOC-biotin)

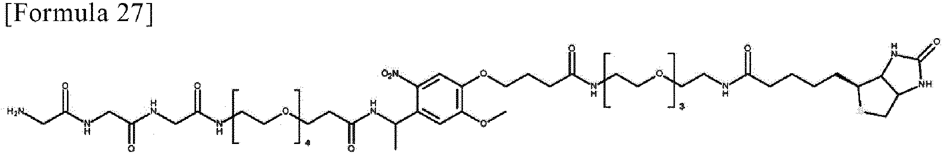

G3-NVOC-biotin was synthesized according to the following scheme.

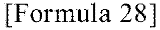

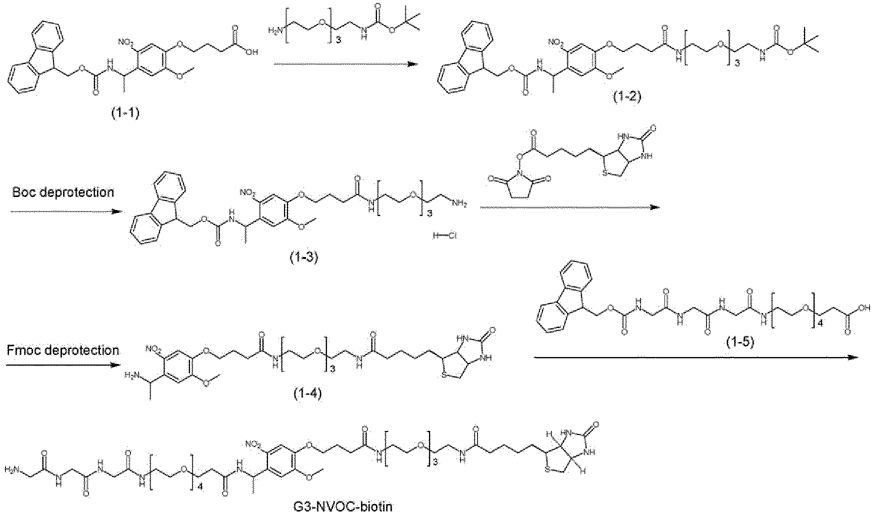

### Synthesis of (9H-fluoren-9-yl)methyl (1-(4-((2,2-dimethyl-4,18-dioxo-3,8,11,14-tetraoxa-5,17-diazahenicosan-21-yl)oxy)-5-methoxy-2-nitrophenyl)ethyl)carbamate (Compound 1-2)

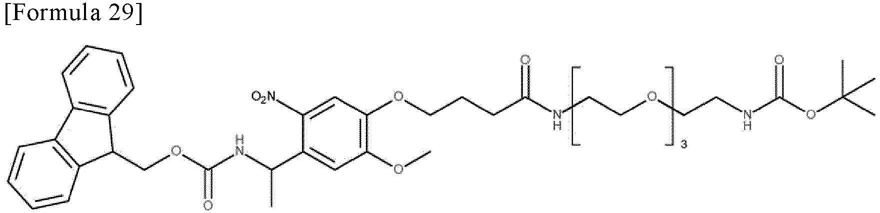

The Compound 1-1 ("Fmoc-Photo-Linker" (manufactured by Iris Biotech), 78.0 mg, 0.15 mmol) was dissolved in NMP (2.7 mL), cooled to 0°C, then HATU (54.6 mg, 0.14 mmol) and DIPEA (25.1 µL, 0.14 mmol) were added thereto. To the mixture was added tert-butyl (2-(2-(2-(2-aminoethoxy)ethoxy)ethoxy)ethyl)carbamate (40.0 mg, 0.14 mmol) dissolved in NMP (0.5 mL) and the mixture was stirred at room temperature for 3 hours. The reaction solution was directly purified by reverse-phase silica gel chromatography (0.1% aqueous formic acid solution/0.1% formic acid in acetonitrile) to obtain the title compound (Compound 1-2, 49.6 mg, 46%).
LCMS (ESI) m/z = 795.4 (M+H)⁺
Retention time: 0.87 minutes (analysis condition SQDFA05)

### Synthesis of (9H-fluoren-9-yl)methyl (1-(4-((1-amino-13-oxo-3,6,9-trioxa-12-azahexadecan-16-yl)oxy)-5-methoxy-2-nitrophenyl)ethyl)carbamate hydrochloride

### (Compound 1-3)

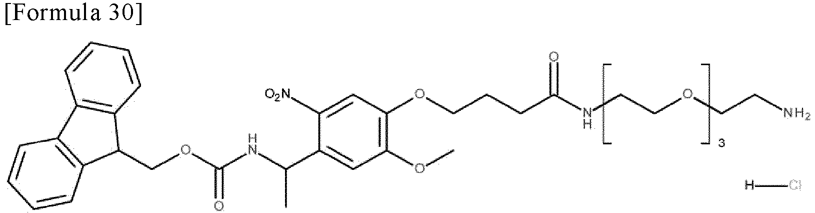

The Compound 1-2 obtained in the previous step was dissolved in DCM (430 µL), to which a 4 molar hydrochloric acid-dioxane solution (430 µL) was added, and the mixture was stirred at room temperature for 20 minutes. The reaction mixture was concentrated under reduced pressure and the obtained residue was used in the next step without purification.
LCMS (ESI) m/z = 695.4 (M+H)⁺
Retention time: 0.60 minutes (analysis condition SQDFA05)

### Synthesis of N-(16-(4-(1-aminoethyl)-2-methoxy-5-nitrophenoxy)-13-oxo-3,6,9-trioxa-12-azahexadecyl)-5-((3aS,4S,6aR)-2-oxohexahydro-1H-thieno[3,4-d]imidazol-4-yl)pentanamide (Compound 1-4)

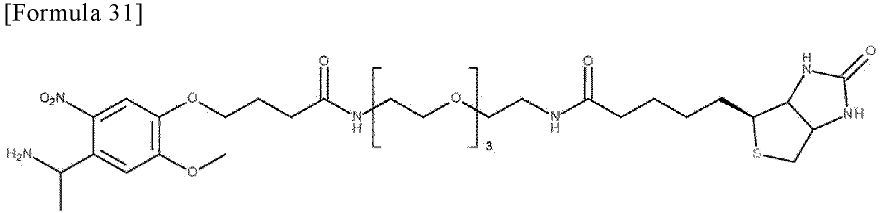

The Compound 1-3 (31.5 mg, 0.043 mmol) obtained in the previous step and (+)-biotin N-hydroxysuccinimide ester (17.7 mg, 0.052 mmol) were dissolved in NMP (1.7 mL), to which DIPEA (22.6 µL, 0.129 mmol) was added, and the mixture was stirred at room temperature for 30 minutes. DBU (13.0 µL, 0.086 mmol) was added to the reaction solution and the mixture was stirred at room temperature for 30 minutes. The reaction solution was acidified by adding a 50% aqueous FA solution, and then purified by reverse-phase silica gel chromatography (0.1% aqueous formic acid solution/0.1% formic acid in acetonitrile) to obtain the title compound (Compound 1-4, 27.9 mg, 93%).
LCMS (ESI) m/z = 699.4 (M+H)⁺
Retention time: 0.38 minutes (analysis condition SQDFA05)

### Synthesis of 1-(9H-fluoren-9-yl)-3,6,9,12-tetraoxo-2,16,19,22,25-pentaoxa-4,7,10,13-tetraazaoctacosan-28-acid (Compound 1-5)

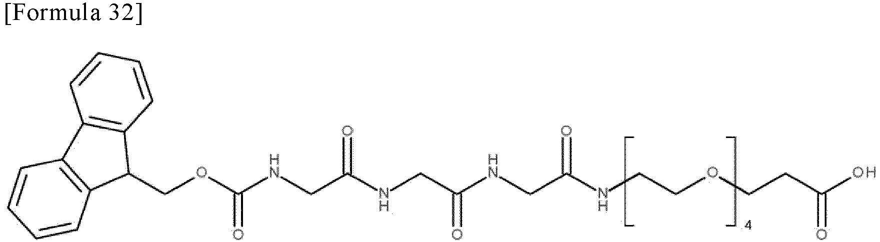

(((9H-fluoren-9-yl)methoxy)carbonyl)glycylglycylglycine (glycylglycylglycine (manufactured by Tokyo Chemical Industry Co., Ltd.) to which the protective group ((9H-fluoren-9-yl)methoxy)carbonyl group is bound by a general method, 100 mg, 0.24 mmol) was dissolved in NMP (4.4 mL) and cooled to 0°C. Then, HOSu (26.7 mg, 0.23 mmol) and DIC (36.1 µL, 0.23 mmol) were added thereto and the mixture was stirred overnight at room temperature. To the reaction solution, 1-amino-3,6,9,12-tetraoxapentadecan-15-acid (58.6 mg, 0.22 mmol) and DIPEA (193.0 µL, 1.11 mmol) were added and the mixture was stirred at room temperature for 3 hours. The reaction solution was acidified by adding a 50% aqueous FA solution, and then purified by reverse-phase silica gel chromatography (0.1% aqueous formic acid solution/0.1% formic acid in acetonitrile) to obtain the title compound (Compound 1-5, 80.5 mg, 55%).
LCMS (ESI) m/z = 659.4 (M+H)⁺
Retention time: 0.58 minutes (analysis condition SQDFA05)

### Synthesis of 1-(2-(2-(2-aminoacetamido)acetamido)acetamido)-N-(1-(4-((4,18-dioxo-22-((3aS,4S,6aR)-2-oxohexahydro-1H-thieno[3,4-d]imidazol-4-yl)-8,11,14-trioxa-5,17-diazadocosyl)oxy)-5-methoxy-2-nitrophenyl)ethyl)-3,6,9,12-tetraoxapentadecan-15-amide (G3-NVOC-biotin)

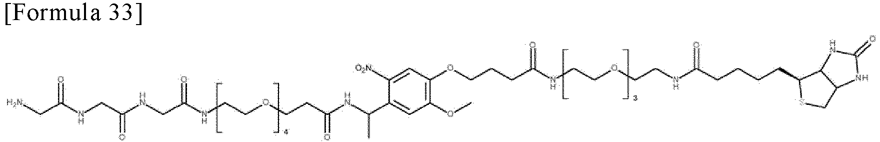

The Compound 1-5 (15.6 mg, 0.024 mmol) obtained in the previous step was dissolved in NMP (429 µL), and cooled to 0°C. Then, HOSu (2.7 mg, 0.024 mmol) and DIC (3.7 µL, 0.024 mmol) were added thereto, and the mixture was stirred overnight at room temperature. To the reaction solution, the Compound 1-4 (15.0 mg, 0.021 mmol) and DIPEA (11.3 µL, 0.064 mmol) were added and the mixture was stirred at room temperature for 5 hours. 2% DBU in DMF (324 µL, 0.043 mmol) was added and the mixture was stirred at room temperature for 30 minutes. The reaction mixture was directly purified by reverse-phase silica gel chromatography (0.1% aqueous formic acid solution/0.1% formic acid in acetonitrile) to obtain the title compound (Compound G3 -NVOC-biotin, 5.0 mg, 21%).
LCMS (ESI) m/z = 1117.1 (M+H)⁺
Retention time: 0.41 minutes (analysis condition SQDFA05)

### Example 1-2: Synthesis of G3-NVOC2-biotin

### Synthesis of 1-(4-(4-(1-amino-2,5,8,24-tetraoxo-12,15,18,21-tetraoxa-3,6,9,25-tetraazaheptacosan-26-yl)-2-methoxy-5-nitrophenoxy)butanamido)-N-(1-(4-((4,18-dioxo-22-((3aS,4S,6aR)-2-oxohexahydro-1H-thieno[3,4-d]imidazol-4-yl)-8,11,14-trioxa-5,17-diazadocosyl)oxy)-5-methoxy-2-nitrophenyl)ethyl)-3,6,9,12-tetraoxapentadecan-15-amide (G3-NVOC2-biotin)

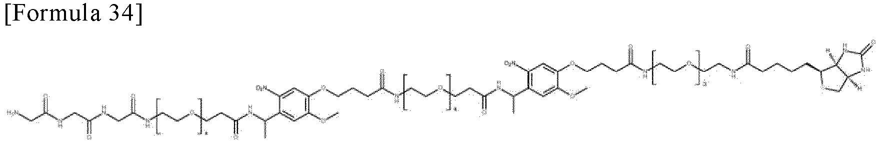

G3-NVOC2-biotin was synthesized according to the following scheme.

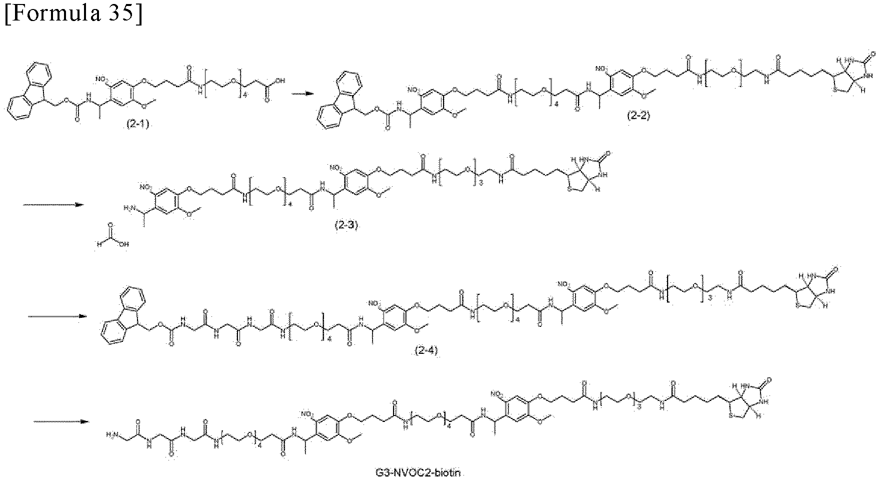

### Synthesis of 20-(4-(1-((((9H-fluoren-9-yl)methoxy)carbonyl)amino)ethyl)-2-methoxy-5-nitrophenoxy)-17-oxo-4,7,10,13-tetraoxa-16-azaicosanoic acid (Compound 2-1)

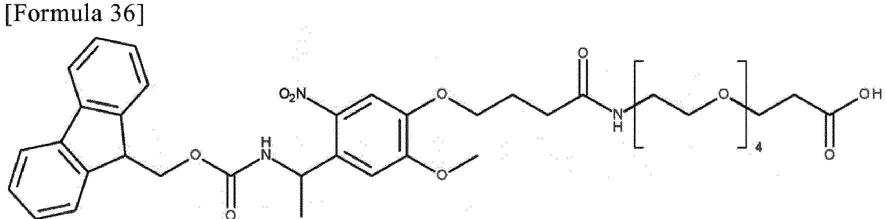

The Compound 1-1 (96.0 mg, 0.184 mmol) was dissolved in NMP (2.5 mL), and cooled to 0°C. Then, HOSu (14.9 mg, 0.129 mmol) and DIC (20.1 µL, 0.129 mmol) were added thereto, and the mixture was stirred overnight at room temperature. To the reaction solution, 1-amino-3,6,9,12-tetraoxapentadecan-15-acid (32.6 mg, 0.123 mmol) and DIPEA (86.0 µL, 0.492 mmol) were added and the mixture was stirred at room temperature for 1 hour. The reaction solution was acidified by adding a 50% aqueous FA solution, and then purified by reverse-phase silica gel chromatography (0.1% aqueous formic acid solution/0.1% formic acid in acetonitrile) to obtain the title compound (Compound 2-1, 48.2 mg, 51%).
LCMS (ESI) m/z = 768.4 (M+H)⁺
Retention time: 0.75 minutes (analysis condition SQDFA05)

### Synthesis of (9H-fluoren-9-yl)methyl (1-(4-((2-(4-((4,18-dioxo-22-((3aS,4S,6aR)-2-oxohexahydro-1H-thieno[3,4-d]imidazole-4-yl)-8,11,14-trioxa-5,17-diazadocosyl)oxy)-5-methoxy-2-nitrophenyl)-4,20-dioxo-7,10,13,16-tetraoxa-3,19-diazatricosan-23-yl)oxy)-5-methoxy-2-nitrophenyl)ethyl)carbamate (Compound 2-2)

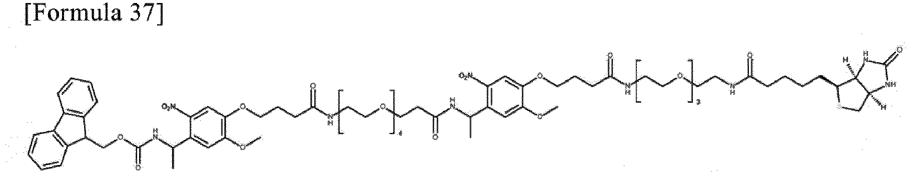

The Compound 2-1 (18.1 mg, 0.024 mmol) obtained in the previous step was dissolved in NMP (429 µL), and cooled to 0°C. Then, HOSu (2.7 mg, 0.024 mmol) and DIC (3.7 µL, 0.024 mmol) were added thereto, and the mixture was stirred overnight at room temperature. To the reaction solution, the Compound 1-4 (15.0 mg, 0.021 mmol) obtained in Example 1-1 and DIPEA (18.7 µL, 0.107 mmol) were added and the mixture was stirred at room temperature for 4 hours. The reaction solution was acidified by adding a 50% aqueous FA solution, and then purified by reverse-phase silica gel chromatography (0.1% aqueous formic acid solution/0.1% formic acid in acetonitrile) to obtain the title compound (Compound 2-2, 12.8 mg, 41%).
LCMS (ESI) m/z = 1448.7 (M+H)⁺
Retention time: 0.74 minutes (analysis condition SQDFA05)

### Synthesis of 1-(4-(4-(1-aminoethyl)-2-methoxy-5-nitrophenoxy)butanamido)-N-(1-(4-((4,18-dioxo-22-((3aS,4S,6aR)-2-oxohexahydro-1H-thieno[3,4-d]imidazol-4-yl)-8,11,14-trioxa-5,17-diazadocosyl)oxy)-5-methoxy-2-nitrophenyl)ethyl)-3,6,9,12-tetraoxapentadecan-15-amide formate (Compound 2-3)

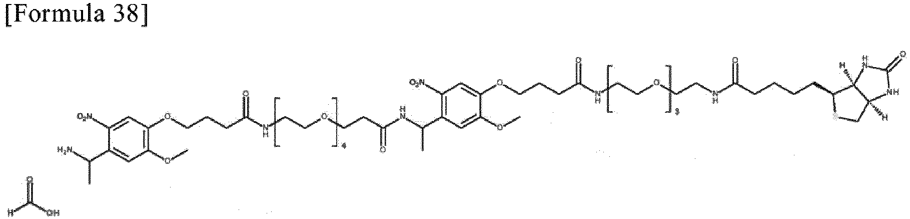

2% DBU in DMF (333 µL) was added to the Compound 2-2 (12.8 mg, 8.84 µmol) obtained in the previous step and the mixture was stirred at room temperature for 30 minutes. The reaction solution was acidified by adding a 50% aqueous FA solution, and then purified by reverse-phase silica gel chromatography (0.1% aqueous formic acid solution/0.1% formic acid in acetonitrile) to obtain the title compound (Compound 2-3, 11.3 mg, 100%).
LCMS (ESI) m/z = 1226.6 (M+H)⁺
Retention time: 0.45 minutes (analysis condition SQDFA05)

### Synthesis of (9H-fluoren-9-yl)methyl (26-(4-((2-(4-((4,18-dioxo-22-((3aS,4S,6aR)-2-oxohexahydro-1H-thieno[3,4-d]imidazole-4-yl)-8,11,14-trioxa-5,17-diazadocosyl)oxy)-5-methoxy-2-nitrophenyl)-4,20-dioxo-7,10,13,16-tetraoxa-3,19-diazatricosan-23-yl)oxy)-5-methoxy-2-nitrophenyl)-2,5,8,24-tetraoxo-12,15,18,21-tetraoxa-3,6,9,25-tetraazaheptacosyl)carbamate (Compound 2-4)

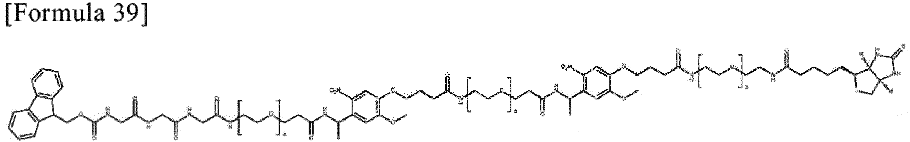

The Compound 1-5 (13.3 mg, 0.020 mmol) obtained in Example 1-1 and HOSu (2.3 mg, 0.020 mmol) were dissolved in NMP (201 µL), and cooled to 0°C. Then, DIC (2.5 mg, 0.020 mmol) was added thereto, and the mixture was stirred overnight at room temperature. To the reaction solution, the Compound 2-3 (12.8 mg, 10.1 µmol) obtained in the previous step and DIPEA (3.9 mg, 0.030 mmol) were added and the mixture was stirred at room temperature for 1 hour. The reaction solution was acidified by adding a 50% aqueous FA solution, and then purified by reverse-phase silica gel chromatography (0.1% aqueous formic acid solution/0.1% formic acid in acetonitrile) to obtain the Compound 2-4 (10.0 mg, 53%).
LCMS (ESI) m/z = 1867.0 (M+H)⁺
Retention time: 0.63 minutes (analysis condition SQDFA05)

### Synthesis of 1-(4-(4-(1-amino-2,5,8,24-tetraoxo-12,15,18,21-tetraoxa-3,6,9,25-tetraazaheptacosan-26-yl)-2-methoxy-5-nitrophenoxy)butanamido)-N-(1-(4-((4,18-dioxo-22-((3aS,4S,6aR)-2-oxohexahydro-1H-thieno[3,4-d]imidazol-4-yl)-8,1 1,14-trioxa-5,17-diazadocosyl)oxy)-5-methoxy-2-nitrophenyl)ethyl)-3,6,9,12-tetraoxapentadecan-15-amide (G3-NVOC2-biotin)

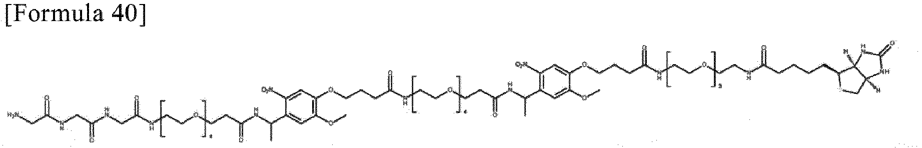

2% DBU in DMF (404 µL, 0.054 mmol) was added to the Compound 2-4 (10.0 mg, 0.020 mmol) obtained in the previous step and the mixture was stirred at room temperature for 15 minutes. The reaction solution was acidified by adding a 50% aqueous FA solution, and then purified by reverse-phase silica gel chromatography (0.1% aqueous formic acid solution/0.1% formic acid in acetonitrile) to obtain the title compound (G3 - NVOC2-biotin, 3.4 mg, 39%).
LCMS (ESI) m/z = 1645.9 (M+H)⁺
Retention time: 0.47 minutes (analysis condition SQDFA05)

### Example 1-3: Synthesis of G3-NVOC3-biotin

### Synthesis of N2-(2-(2-(2-(4-(4-(16-(4-(16-(4-(1-(2-(2-(2-aminoacetamido)acetamido)acetamido)ethyl)-2-methoxy-5-nitrophenoxy)-4,13-dioxo-6,9-dioxa-3,12-diazahexadecan-2-yl)-2-methoxy-5-nitrophenoxy)-4,13-dioxo-6,9-dioxa-3,12-diazahexadecan-2-yl)-2-methoxy-5-nitrophenoxy)butanamido)ethoxy)ethoxy)acetyl)-N6-(5-((3aS,4S,6aR)-2-oxohexahydro-1H-thieno[3,4-d]imidazol-4-yl)pentanoyl)-L-lysine compound with 2,2,2-trifluoroacetic acid (G3-NVOC3-biotin)

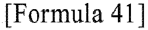

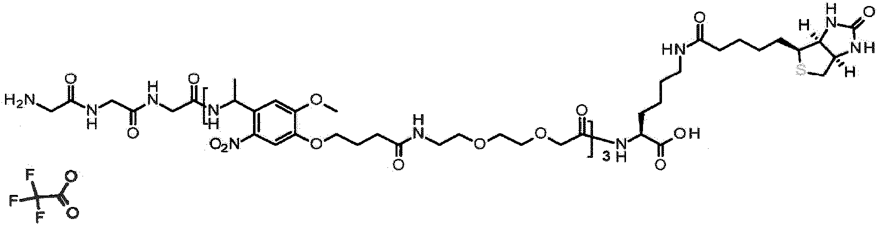

Solid-phase synthesis was performed by the following route according to the peptide synthesis method by the Fmoc method described in the literature. That is, it has four steps: 1) peptide elongation reaction by the Fmoc method from the N-terminus of a carboxylic acid of the C-terminal amino acid supported on a 2-chlorotrityl resin, 2) a process of excising the peptide from the 2-chlorotrityl resin, 3) deprotection of the N-terminal Fmoc group, and 4) purification by column chromatography.

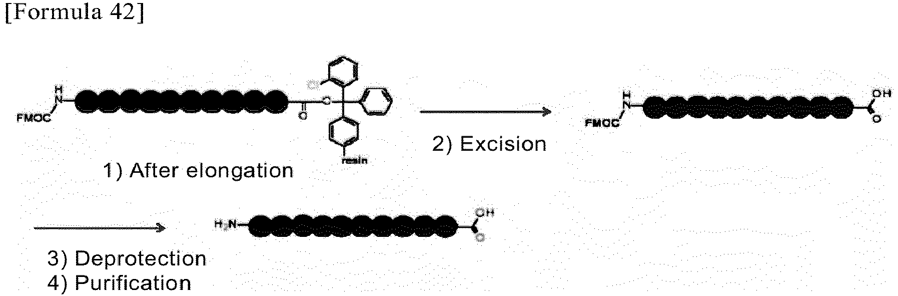

The following Fmoc-protected compounds were used for the synthesis of G3-NVOC3-biotin.

**[Table 2]**

| **Name** | **Structure** |
|---|---|
| Fmoc-Biocytin-OH | 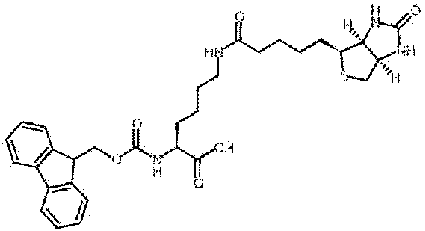 |
| Fmoc-PEG-OH | 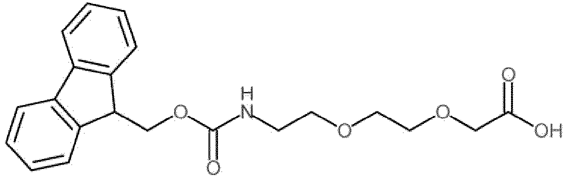 |
| Fmoc-Photo-Linker | 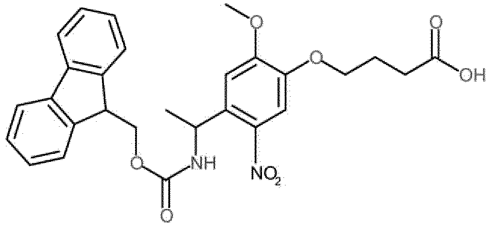 |
| Fmoc-Gly-OH | 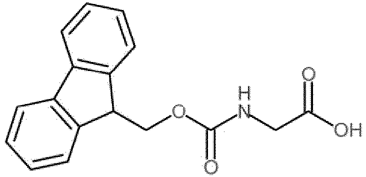 |

### G3-NVOC3-biotin Sequence

**[Table 3]**

| | | | | | | | | | | | |
|---|---|---|---|---|---|---|---|---|---|---|---|
| N-term | 10 | 9 | 8 | 7 | 6 | 5 | 4 | 3 | 2 | 1 | C-term |
| NH₂ | Gly | Gly | Gly | Photo-Linker | PEG | Photo-Linker | PEG | Photo-Linker | PEG | Biocytin | OH |

### Synthesis of N2-((9H-fluoren-9-yl)methoxy)carbonyl)-N6-(5-((3aS,4S,6aR)-2-oxohexahydro-1H-thieno[3,4-d]imidazol-4-yl)pentanoyl)-L-lysine-2-chlorotrityl resin (Compound 3-1)

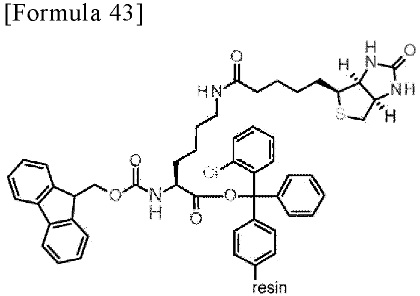

A reaction vessel with a filter was charged with 2-chlorotrityl chloride resin (1.60 mmol/g, 100 to 200 mesh, 1% DVB, purchased from Watanabe Chemical Industries, Ltd., 2.62 g, 8.0 mmol) and water-free DCM (25.0 mL), and the mixture was shaken at room temperature for 30 minutes. Nitrogen pressure was applied to remove DCM, water-free DCM (25.0 mL) was further added, and then nitrogen pressure was applied to remove DCM. A mixed solution of N2-(((9H-fluoren-9-yl)methoxy)carbonyl)-N6-(5-((3aS,4S,6aR)-2-oxohexahydro-1H-thieno[3,4-d]imidazol-4-yl)pentanoyl)-L-lysine (1.67 g, 2.8 mmol), water-free MeOH (1.3 mL) and DIPEA (3.3 mL) dissolved in water-free DCM (27.0 mL) was added to the reaction vessel and shaken at room temperature for 1 hour. Nitrogen pressure was applied to remove the reaction solution, a mixed solution obtained by adding water-free MeOH (5.0 mL) and DIPEA (3.3 mL) to water-free DCM (40.0 mL) was then added in the reaction vessel, and the mixture was shaken for 2 hours. Nitrogen pressure was applied to remove the reaction solution, water-free DCM (40.0 mL) was then put in the reaction vessel, and nitrogen pressure was applied to remove the reaction solution. The washing of the resin with DCM was repeated twice, and the obtained resin was dried overnight under reduced pressure to obtain N2-((9H-fluoren-9-yl)methoxy)carbonyl)-N6-(5-((3aS,4S,6aR)-2-oxohexahydro-1H-thieno[3,4-d]imidazol-4-yl)pentanoyl)-L-lysine-2-chlorotrityl resin (Compound 3-1, 5.6 g).

The obtained Compound 3-1 (10.2 mg) was put in the reaction vessel, DMF (2.0 mL) was added, and the resin was swollen at room temperature for 1 hour. Then, DBU (0.04 mL) was added and the mixture was shaken at 30°C for 30 minutes. Then, DMF was added to the reaction mixed solution until reaching 10 mL, to 1.0 mL taken therefrom was added 11.5 mL of DMF, and its absorbance (294 nm) was measured (measured using an ultra violet-visible spectrophotometer UV-1800 (manufactured by Shimadzu Corporation, cell length 1.0 cm)), from which the loading amount of Compound 3 -1 was calculated to be 0.3515 mmol/g.

### Synthesis of N2-(2-(2-(2-(4-(4-(16-(4-(16-(4-(1-(2-(2-(2-((((9H-fluoren-9-yl)methoxy)carbonyl)amino)acetamido)acetamido)acetamido)ethyl)-2-methoxy-5-nitrophenoxy)-4,13-dioxo-6,9-dioxa-3,12-diazahexadecan-2-yl)-2-methoxy-5-nitrophenoxy)-4,13-dioxo-6,9-dioxa-3,12-diazahexadecan-2-yl)-2-methoxy-5-nitrophenoxy)butanamido)ethoxy)ethoxy)acetyl)-N6-(5-((3aS,4S,6aR)-2-oxohexahydro-1H-thieno[3,4-d]imidazol-4-yl)pentanoyl)-L-lysine (Fmoc-protected form of G3-NVOC3-biotin)

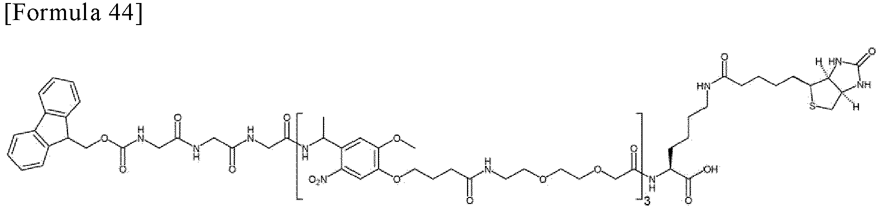

### Synthetic Sequence

**[Table 4]**

| | | | | | | | | | | | |
|---|---|---|---|---|---|---|---|---|---|---|---|
| N-term | 10 | 9 | 8 | 7 | 6 | 5 | 4 | 3 | 2 | 1 | C-term |
| Fmoc | Gly | Gly | Gly | Photo-Linker | PEG | Photo-Linker | PEG | Photo-Linker | PEG | Biocytin | OH |

### 1) Solid-phase synthesis using automated synthesizer

Solid-phase synthesis by the Fmoc method was performed using a peptide synthesizer (Multipep RS; manufactured by Intavis) according to the method described in WO 2013/100132. Detailed procedures of operations were consistent with those in the manual attached to the synthesizing machine.

The Compound 3-1 (100 mg per column, 10 columns) obtained in the previous step, an NMP solution of the Fmoc-protected compounds listed in Table 2 (0.6 mol/L) and 1-hydroxy-7-azabenzotriazole (HOAt), and an N,N-dimethylformamide (DMF) solution (10% v/v) of diisopropylcarbodiimide (DIC) were set in the synthesizer.

The synthesis was performed using a DMF solution of DBU (2% v/v) as the Fmoc deprotection solution. The resin was washed with DMF, then after Fmoc deprotection, the condensation reaction of the Fmoc amino acid was set to one cycle, and this cycle was repeated to elongate a peptide on the resin surface. After completing peptide elongation, the resin was washed with DMF and DCM on the synthesizer.

### 2) Excision of elongated peptide from resin

After DCM was added to the chain peptides supported on the solid phase obtained by the above method with the method described in WO2013/100132, and the resin was reswelled, 2,2,2-trifluoroethanol (TFE)/DCM (1/1, v/v, 2 mL) was added to the resin and the mixture was shaken at room temperature for 2 hours. The solution in the tube was then filtered through a column for synthesis to remove the resin, and the remaining resin was further washed twice with 2,2,2-trifluoroethanol (TFE)/DCM (1/1, v/v, 1 mL). All the obtained isolating solutions were combined and concentrated under reduced pressure. The obtained residue was used in the next step without purification.

### 3) Deprotection of N-terminal Fmoc group, 4) Purification by column chromatography

The crude product obtained in the previous step, N2-(2-(2-(2-(4-(4-(16-(4-(16-(4-(1-(2-(2-(2-((((9H-fluorene-9-yl)methoxy)carbonyl)amino)acetamido)acetamido)acetamido)ethyl)-2-methoxy-5-nitrophenoxy)-4,13-dioxo-6,9-dioxa-3,12-diazahexadecan-2-yl)-2-methoxy-5-nitrophenoxy)-4,13-dioxo-6,9-dioxa-3,12-diazahexadecan-2-yl)-2-methoxy-5-nitrophenoxy)butanamido)ethoxy)ethoxy)acetyl)-N6-(5-((3aS,4S,6aR)-2-oxohexahydro-1H-thieno[3,4-d]imidazol-4-yl)pentanoyl)-L-lysine (155.9 mg, 0.076 mmol) and DBU (23.0 µL, 0.153 mmol) were dissolved in DMF (7.6 mL) and stirred at room temperature for 5 hours. The reaction solution was cooled with ice and TFA (17.6 µL, 0.229 mmol) was added. The obtained mixed solution was purified by reverse-phase silica gel chromatography (0.05% aqueous TFA solution/0.05% TFA in acetonitrile) to obtain the title compound (G3-NVOC3-biotin, 83 mg, 56%).
LCMS (ESI) m/z = 1821.54 (M+H)⁺
Retention time: 0.53 minutes (analysis condition SQDFA05)

### Example 1-4: Synthesis of G3-NVOC4-biotin

### Synthesis of N2-(2-(2-(2-(4-(4-(16-(4-(16-(4-(16-(4-(1-(2-(2-(2-aminoacetamido)acetamido)acetamido)ethyl)-2-methoxy-5-nitrophenoxy)-4,13-dioxo-6,9-dioxa-3,12-diazahexadecan-2-yl)-2-methoxy-5-nitrophenoxy)-4,13-dioxo-6,9-dioxa-3,12-diazahexadecan-2-yl)-2-methoxy-5-nitrophenoxy)-4,13-dioxo-6,9-dioxa-3,12-diazahexadecan-2-yl)-2-methoxy-5-nitrophenoxy)butanamido)ethoxy)ethoxy)acetyl)-N6-(5-((3aS,4S,6aR)-2-oxohexahydro-1H-thieno[3,4-d]imidazol-4-yl)pentanoyl)-L-lysine compound with 2,2,2-trifluoroacetic acid (Synthesis of G3-NVOC4-biotin)

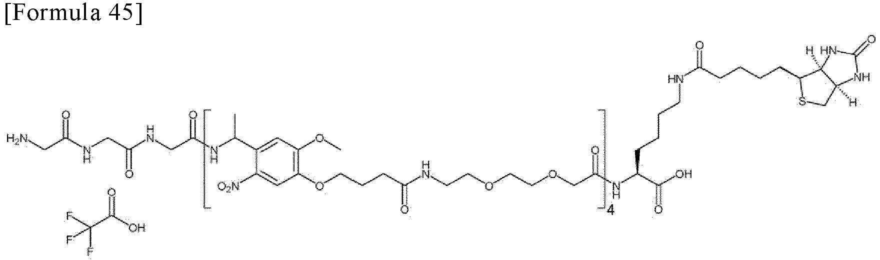

The synthesis was performed according to a general method of peptide solid-phase synthesis.
LCMS (ESI) m/z = 2247.05 (M+H)⁺
Retention time: 0.53 minutes (analysis condition SQDFA05)

### Example 1-5: Synthesis of G3-CMRN3-biotin

### Synthesis of tert-butyl 4-((6-bromo-4-(hydroxymethyl)-2-oxo-2H-chromen-7-yl)oxy)butanoate (Compound 5-1)

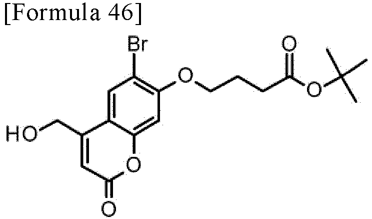

Under nitrogen atmosphere, DMSO (7.00 mL) was added to 6-bromo-7-hydroxy-4-(hydroxymethyl)coumarin (0.949 g, 3.50 mmol) at room temperature conditions, then N,N-diisopropylethylamine (DIPEA) (1.83 mL, 10.5 mmol) and tert-butyl 4-bromobutanoate (1.25 mL, 7.00 mmol) were added. The reaction mixture was stirred at 70°C for 6 hours. The reaction mixture was cooled to room temperature, then purified by reverse-phase silica gel column chromatography (0.1% aqueous formic acid solution/0.1% solution of formic acid in acetonitrile) to obtain tert-butyl 4-((6-bromo-4-(hydroxymethyl)-2-oxo-2H-chromen-7-yl)oxy)butanoate (Compound 5-1) (1.31 g, 91%).
LCMS (ESI) m/z = 413.2 (M+H)⁺
Retention time: 0.85 minutes (analysis condition SQDFA05)

### Synthesis of tert-butyl 4-((4-(13-(9H-fluoren-9-yl)-3,11-dioxo-2,7,12-trioxa-4,10-diazatridecyl)-6-bromo-2-oxo-2H-chromen-7-yl)oxy)butanoate (Compound 5-2)

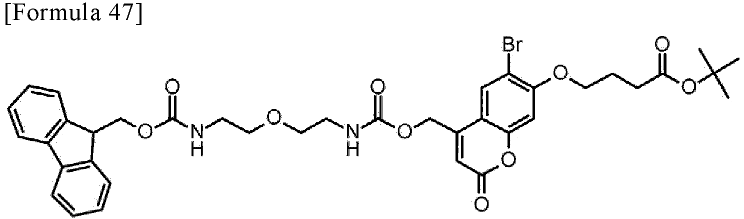

Under nitrogen atmosphere, tert-butyl 4-((6-bromo-4-(hydroxymethyl)-2-oxo-2H-chromen-7-yl)oxy)butanoate (Compound 5-1) (1.31 g, 3.17 mmol) was dissolved in acetonitrile (7.92 mL), to which pyridine (0.513 mL, 6.34 mmol) and 4-nitrophenyl chloroformate (0.767 g, 3.80 mmol) were added, and the mixture was stirred at room temperature for 70 minutes. To the reaction mixture was further added N,N-diisopropylethylamine (DIPEA) (1.33 mL, 7.61 mmol) and (9H-fluoren-9-yl)methyl (2-(2-aminoethoxy)ethyl)carbamate hydrochloride (1.50 g, 4.12 mmol). The reaction mixture was stirred at room temperature for 2 hours, then 1 M hydrochloric acid was added, and the mixture was purified by reverse-phase silica gel column chromatography (0.1% aqueous formic acid solution/0.1% solution of formic acid in acetonitrile) to obtain tert-butyl 4-((4-(13-(9H-fluoren-9-yl)-3,11-dioxo-2,7,12-trioxa-4,10-diazatridecyl)-6-bromo-2-oxo-2H-chromen-7-yl)oxy)butanoate (Compound 5-2) (2.28 g, 94%).
LCMS (ESI) m/z = 765.4 (M+H)⁺
Retention time: 1.06 minutes (analysis condition SQDFA05)

### Synthesis of 4-((4-(13-(9H-fluoren-9-yl)-3,11-dioxo-2,7,12-trioxa-4,10-diazatridecyl)-6-bromo-2-oxo-2H-chromen-7-yl)oxy)butanoic acid (Compound 5-3)

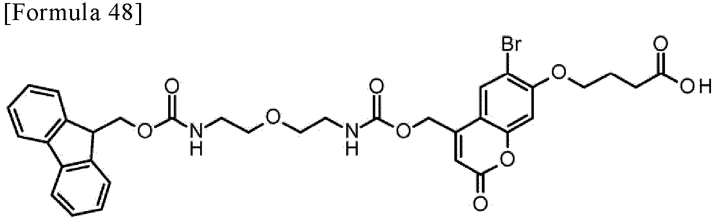

A 4N hydrochloric acid/1,4-dioxane solution (40 mL) was added to tert-butyl 4-((4-(13-(9H-fluoren-9-yl)-3,11-dioxo-2,7,12-trioxa-4,10-diazatridecyl)-6-bromo-2-oxo-2H-chromen-7-yl)oxy)butanoate (Compound 5-2) (2.28 g, 2.98 mmol), and the mixture was stirred at room temperature for 5 hours. The reaction solution was concentrated under reduced pressure to obtain 4-((4-(13-(9H-fluoren-9-yl)-3,11-dioxo-2,7,12-trioxa-4,10-diazatridecyl)-6-bromo-2-oxo-2H-chromen-7-yl)oxy)butanoic acid (Compound 5-3) (2.11 g).
LCMS (ESI) m/z = 709.3 (M+H)⁺
Retention time: 0.82 minutes (analysis condition SQDFA05)

### Synthesis of N2-(2-(2-(2-(4-((4-(23-((4-(23-((4-(12-(2-(2-aminoacetamido)acetamido)-3,11-dioxo-2,7-dioxa-4,10-diazadodecyl)-6-bromo-2-oxo-2H-chromen-7-yl)oxy)-3,11,20-trioxo-2,7,13,16-tetraoxa-4,10,19-triazatricosyl)-6-bromo-2-oxo-2H-chromen-7-yl)oxy)-3,11,20-trioxo-2,7,13,16-tetraoxa-4,10,19-triazatricosyl)-6-bromo-2-oxo-2H-chromen-7-yl)oxy)butanamido)ethoxy)ethoxy)acetyl)-N6-(5-((3aS,4S,6aR)-2-oxohexahydro-1H-thieno[3,4-d]imidazol-4-yl)pentanoyl)-L-lysine (Compound 5-4: G3-CMRN3-biotin)

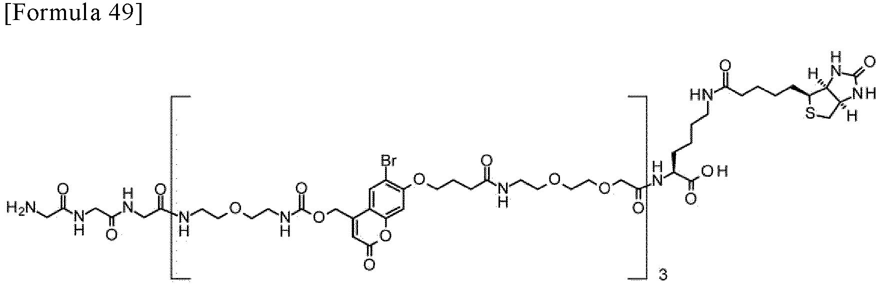

Similar to the synthesis of G3-NVOC3-biotin (Example 1-3), peptide solid-phase synthesis by the Fmoc method was performed using a peptide synthesizer (Multipep RS, manufactured by CEM). The Fmoc-protected compound was purified by reverse-phase silica gel column chromatography (0.1% aqueous formic acid solution/0.1% solution of formic acid in acetonitrile) using the Compound 5-3 instead of Fmoc-Photo-Linker to obtain N2-(2-(2-(2-(4-((4-(23-((4-(23-((4-(12-(2-(2-aminoacetamido)acetamido)-3,1 1-dioxo-2,7-dioxa-4,10-diazadodecyl)-6-bromo-2-oxo-2H-chromen-7-yl)oxy)-3,11,20-trioxo-2,7,13,16-tetraoxa-4,10,19-triazatricosyl)-6-bromo-2-oxo-2H-chromen-7-yl)oxy)-3,11,20-trioxo-2,7,13,16-tetraoxa-4,10,19-triazatricosyl)-6-bromo-2-oxo-2H-chromen-7-yl)oxy)butanamido)ethoxy)ethoxy)acetyl)-N6-(5-((3aS,4S,6aR)-2-oxohexahydro-1H-thieno[3,4-d]imidazol-4-yl)pentanoyl)-L-lysine (Compound 5-4: G3-CMRN3-biotin).
LCMS (ESI) m/z = 2383.7 (M+H)⁺
Retention time: 0.51 minutes (analysis condition SQDFA05)

### Comparative Example 1: Synthesis of G3K-biotin

### Synthesis of N2-glycylglycylglycyl-N6-(5-((3aS,4S,6aR)-2-oxohexahydro-1H-thieno[3,4-d]imidazol-4-yl)pentanoyl)-L-lysine (G3K-biotin)

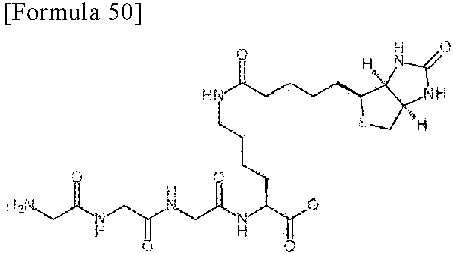

(((9H-fluoren-9-yl)methoxy)carbonyl)glycylglycylglycyl-L-lysine compound with 2,2,2-trifluoroacetic acid (12 mg, 0.018 mmol) and DIPEA (13.4 µL, 0.076 mmol) were dissolved in DMSO (600 µL), and the mixture was stirred at room temperature for 1 hour. 2% DBU in DMF (231 µL) was added to the reaction solution and the mixture was stirred overnight at room temperature. The obtained mixture was purified by preparative-HPLC to obtain G3K-biotin (7.6 mg, 91%).
LCMS (ESI) m/z = 544.62 (M+H)⁺
Retention time: 0.28 minutes (analysis condition SQDFA05)

### Synthesis Example 1: Preparation of target proteins used for photocleavage experiments and panning

GTPase KRas (KRAS), Nutilisation substance protein A (NusA), Glutathione S-transferase (GST) and Superfolder green fluorescent protein (sfGFP) were used as target proteins used for photocleavage experiments and panning.

### Synthesis Example 2-1: Preparation of Sortase A pentamutant (eSrtA)

eSrtA was expressed in *E. coli* and purified in accordance with the method in Non Patent Literature Proc. Natl. Acad. Sci. USA. 2011 Jul 12; 108(28): 11399-11404. The Sortase A synthesized here is not the wild type, but the more active
P94R/D160N/D165A/K190E/K196T pentamutant (Sortase A pentamutant, eSrtA).

eSrtA amino acid sequence (Sequence No. 1):
MQAKPQIPKDKSKVAGYIEIPDADIKEPVYPGPATREQLNRGVSFAEENESLDDQNIS
IAGHTFIDRPNYQFTNLKAAKKGSMVYFKVGNETRKYKMTSIRNVKPTAVEVLDEQ
KGKDKQLTLITCDDYNEETGVWETRKIFVATEVKLEHHHHHH

### Synthesis Example 2-2: Preparation of Sepharose-immobilized eSrtA

Sepharose-immobilized eSrtA was prepared according to the method in Non Patent Literature Nature Protocols, 2015, 10, 508-516.

### Synthesis Example 3: Preparation of Sortase-tagged Target Proteins

The following proteins having a FLAG tag (DYKDDDDK), a sortase tag (LPMTGG), and a His tag (HHHHHHH) at the C-terminus were expressed in *E. coli* and purified.

**[Table 5]**

| **Protein Name** | **Sequence** |
|---|---|
| KRAS4Bwt(C_FL AG-Sor-His) (Sequence No. 2) | |
| NusA-SrtAHis (Sequence No. 3) | |
| GST-SrtAHis (Sequence No. 4) | |
| sfGFP-SrtAHis (Sequence No. 5) | |

### Example 2: Preparation of Target Proteins with Biotin Linkers

The binding reaction of the chemically synthesized biotin linker to the target protein was performed using the eSrtA or Sepharose-immobilized eSrtA synthesized by the method described in Synthesis Example 2-1 or 2-2. eSrtA and Sepharose-immobilized eSrtA are transpeptidases that recognize LPXTG sequences (X is any amino acid).

The sequences of the target proteins with biotin linkers are shown in Tables 6 to 10. The concentration conditions for the biotin linker binding reaction are shown in Table 11.

**[Table 6]**

| **Protein Name** | **Sequence** |
|---|---|
| KRAS-NVOC-biotin | MTEYKLVVVGAGGVGKSALTIQLIQNHFVDEYDPTIEDSYRKQVVIDGETCLLDIL DTAGQEEYSAMRDQYMRTGEGFLCVFAINNTKSFEDIHHYREQIKRVKDSEDVP MVLVGNKCDLPSRTVDTKQAQDLARSYGIPFIETSAKTRQGVDDAFYTLVREIRK HKEKMSKDGGSSDYKDDDDKGGSSLPMT[G3-NVOC-biotin] |
| KRAS-NVOC2-biotin | MTEYKLVVVGAGGVGKSALTIQLIQNHFVDEYDPTIEDSYRKQVVIDGETCLLDIL DTAGQEEYSAMRDQYMRTGEGFLCVFAINNTKSFEDIHHYREQIKRVKDSEDVP MVLVGNKCDLPSRTVDTKQAQDLARSYGIPFIETSAKTRQGVDDAFYTLVREIRK HKEKMSKDGGSSDYKDDDDKGGSSLPMT[G3-NVOC2-biotin] |
| KRAS-NVOC3-biotin | MTEYKLVVVGAGGVGKSALTIQLIQNHFVDEYDPTIEDSYRKQVVIDGETCLLDIL DTAGQEEYSAMRDQYMRTGEGFLCVFAINNTKSFEDIHHYREQIKRVKDSEDVP MVLVGNKCDLPSRTVDTKQAQDLARSYGIPFIETSAKTRQGVDDAFYTLVREIRK HKEKMSKDGGSSDYKDDDDKGGSSLPMT[G3-NVOC3-biotin] |

**[Table 7]**

| **Protein Name** | **Sequence** |
|---|---|
| NusA-NVOC3-biotin | MNKEILAVVEAVSNEKALPREKIFEALESALATATKKKYEQEIDVRVQIDRKSGD FDTFRRWLVVDEVTQPTKEITLEAARYEDESLNLGDYVEDQIESVTFDRITTQTA KQVIVQKVREAERAMVVDQFREHEGEIITGVVKKVNRDNISLDLGNNAEAVILRE DMLPRENFRPGDRVRGVLYSVRPEARGAQLFVTRSKPEMLIELFRIEVPEIGEE VIEIKAAARDPGSRAKIAVKTNDKRIDPVGACVGMRGARVQAVSTELGGERIDIV LWDDNPAQFVINAMAPADVASIVVDEDKHTMDIAVEAGNLAQAIGRNGQNVRL ASQLSGWELNVMTVDDLQAKHQAEAHAAIDTFTKYLDIDEDFATVLVEEGFSTL EELAYVPMKELLEIEGLDEPTVEALRERAKNALATIAQAQEESLGDNKPADDLLN LEGVDRDLAFKLAARGVCTLEDLAEQGIDDLADIEGLTDEKAGALIMAARNICWF GDEAGGSSDYKDDDDKGGSSLPMT[G3-NVOC3-biotin] |
| NusA-biotin | MNKEILAVVEAVSNEKALPREKIFEALESALATATKKKYEQEIDVRVQIDRKSGD FDTFRRWLVVDEVTQPTKEITLEAARYEDESLNLGDYVEDQIESVTFDRITTQTA KQVIVQKVREAERAMVVDQFREHEGEIITGVVKKVNRDNISLDLGNNAEAVILRE DMLPRENFRPGDRVRGVLYSVRPEARGAQLFVTRSKPEMLIELFRIEVPEIGEE VIEIKAAARDPGSRAKIAVKTNDKRIDPVGACVGMRGARVQAVSTELGGERIDIV LWDDNPAQFVINAMAPADVASIVVDEDKHTMDIAVEAGNLAQAIGRNGQNVRL ASQLSGWELNVMTVDDLQAKHQAEAHAAIDTFTKYLDIDEDFATVLVEEGFSTL EELAYVPMKELLEIEGLDEPTVEALRERAKNALATIAQAQEESLGDNKPADDLLN LEGVDRDLAFKLAARGVCTLEDLAEQGIDDLADIEGLTDEKAGALIMAARNICWF GDEAGGSSDYKDDDDKGGSSLPMT[G3K-biotin] |

**[Table 8]**

| **Protein Name** | **Sequence** |
|---|---|
| GST-NVOC-biotin | MSPILGYWKIKGLVQPTRLLLEYLEEKYEEHLYERDEGDKWRNKKFELGLEFPN LPYYIDGDVKLTQSMAIIRYIADKHNMLGGCPKERAEISMLEGAVLDIRYGVSRIA YSKDFETLKVDFLSKLPEMLKMFEDRLCHKTYLNGDHVTHPDFMLYDALDVVLY MDPMCLDAFPKLVCFKKRIEAIPQIDKYLKSSKYIAWPLQGWQATFGGGDHPPK SDGGSSDYKDDDDKGGSSLPMT[G3-NVOC-biotin] |
| GST-NVOC2-biotin | MSPILGYWKIKGLVQPTRLLLEYLEEKYEEHLYERDEGDKWRNKKFELGLEFPN LPYYIDGDVKLTQSMAIIRYIADKHNMLGGCPKERAEISMLEGAVLDIRYGVSRIA YSKDFETLKVDFLSKLPEMLKMFEDRLCHKTYLNGDHVTHPDFMLYDALDVVLY MDPMCLDAFPKLVCFKKRIEAIPQIDKYLKSSKYIAWPLQGWQATFGGGDHPPK SDGGSSDYKDDDDKGGSSLPMT[G3-NVOC2-biotin] |
| GST-NVOC3-biotin | MSPILGYWKIKGLVQPTRLLLEYLEEKYEEHLYERDEGDKWRNKKFELGLEFPN LPYYIDGDVKLTQSMAIIRYIADKHNMLGGCPKERAEISMLEGAVLDIRYGVSRIA YSKDFETLKVDFLSKLPEMLKMFEDRLCHKTYLNGDHVTHPDFMLYDALDVVLY MDPMCLDAFPKLVCFKKRIEAIPQIDKYLKSSKYIAWPLQGWQATFGGGDHPPK SDGGSSDYKDDDDKGGSSLPMT[G3-NVOC3-biotin] |
| GST-NVOC4-biotin | MSPILGYWKIKGLVQPTRLLLEYLEEKYEEHLYERDEGDKWRNKKFELGLEFPN LPYYIDGDVKLTQSMAIIRYIADKHNMLGGCPKERAEISMLEGAVLDIRYGVSRIA YSKDFETLKVDFLSKLPEMLKMFEDRLCHKTYLNGDHVTHPDFMLYDALDVVLY MDPMCLDAFPKLVCFKKRIEAIPQIDKYLKSSKYIAWPLQGWQATFGGGDHPPK SDGGSSDYKDDDDKGGSSLPMT[G3-NVOC4-biotin] |
| GST-biotin | MSPILGYWKIKGLVQPTRLLLEYLEEKYEEHLYERDEGDKWRNKKFELGLEFPN LPYYIDGDVKLTQSMAIIRYIADKHNMLGGCPKERAEISMLEGAVLDIRYGVSRIA YSKDFETLKVDFLSKLPEMLKMFEDRLCHKTYLNGDHVTHPDFMLYDALDVVLY MDPMCLDAFPKLVCFKKRIEAIPQIDKYLKSSKYIAWPLQGWQATFGGGDHPPK SDGGSSDYKDDDDKGGSSLPMT[G3K-biotin] |

**[Table 9]**

| **Protein Name** | **Sequence** |
|---|---|
| sfGFP-NVOC-biotin | MVSKGEELFTGVVPILVELDGDVNGHKFSVRGEGEGDATNGKLTLKFICTTGKLPV PWPTLVTTLTYGVQCFSRYPDHMKRHDFFKSAMPEGYVQERTISFKDDGTYKTRA EVKFEGDTLVNRIELKGIDFKEDGNILGHKLEYNFNSHNVYITADKQKNGIKANFKIR HNVEDGSVQLADHYQQNTPIGDGPVLLPDNHYLSTQSVLSKDPNEKRDHMVLLEF VTAAGITHGMDELYKGGSSDYKDDDDKGGSSLPMT[G3-NVOC-biotin] |
| sfGFP-NVOC2-biotin | MVSKGEELFTGVVPILVELDGDVNGHKFSVRGEGEGDATNGKLTLKFICTTGKLPV PWPTLVTTLTYGVQCFSRYPDHMKRHDFFKSAMPEGYVQERTISFKDDGTYKTRA EVKFEGDTLVNRIELKGIDFKEDGNILGHKLEYNFNSHNVYITADKQKNGIKANFKIR HNVEDGSVQLADHYQQNTPIGDGPVLLPDNHYLSTQSVLSKDPNEKRDHMVLLEF VTAAGITHGMDELYKGGSSDYKDDDDKGGSSLPMT[G3-NVOC2-biotin] |
| sfGFP-NVOC3-biotin | MVSKGEELFTGVVPILVELDGDVNGHKFSVRGEGEGDATNGKLTLKFICTTGKLPV PWPTLVTTLTYGVQCFSRYPDHMKRHDFFKSAMPEGYVQERTISFKDDGTYKTRA EVKFEGDTLVNRIELKGIDFKEDGNILGHKLEYNFNSHNVYITADKQKNGIKANFKIR HNVEDGSVQLADHYQQNTPIGDGPVLLPDNHYLSTQSVLSKDPNEKRDHMVLLEF VTAAGITHGMDELYKGGSSDYKDDDDKGGSSLPMT[G3-NVOC3-biotin] |
| sfGFP-NVOC4-biotin | MVSKGEELFTGVVPILVELDGDVNGHKFSVRGEGEGDATNGKLTLKFICTTGKLPV PWPTLVTTLTYGVQCFSRYPDHMKRHDFFKSAMPEGYVQERTISFKDDGTYKTRA EVKFEGDTLVNRIELKGIDFKEDGNILGHKLEYNFNSHNVYITADKQKNGIKANFKIR HNVEDGSVQLADHYQQNTPIGDGPVLLPDNHYLSTQSVLSKDPNEKRDHMVLLEF VTAAGITHGMDELYKGGSSDYKDDDDKGGSSLPMT[G3-NVOC4-biotin] |
| sfGFP-biotin | MVSKGEELFTGVVPILVELDGDVNGHKFSVRGEGEGDATNGKLTLKFICTTGKLPV PWPTLVTTLTYGVQCFSRYPDHMKRHDFFKSAMPEGYVQERTISFKDDGTYKTRA EVKFEGDTLVNRIELKGIDFKEDGNILGHKLEYNFNSHNVYITADKQKNGIKANFKIR HNVEDGSVQLADHYQQNTPIGDGPVLLPDNHYLSTQSVLSKDPNEKRDHMVLLEF VTAAGITHGMDELYKGGSSDYKDDDDKGGSSLPMT[G3K-biotin] |

**[Table 10]**

| **Protein Name** | **Sequence** |
|---|---|
| GST-CMRN3 -biotin | MSPILGYWKIKGLVQPTRLLLEYLEEKYEEHLYERDEGDKWRNKKFELGLEFPN LPYYIDGDVKLTQSMAIIRYIADKHNMLGGCPKERAEISMLEGAVLDIRYGVSRIA YSKDFETLKVDFLSKLPEMLKMFEDRLCHKTYLNGDHVTHPDFMLYDALDVVLY MDPMCLDAFPKLVCFKKRIEAIPQIDKYLKSSKYIAWPLQGWQATFGGGDHPPK SDGGSSDYKDDDDKGGSSLPMT[G3-CMRN3-biotin] |

**[Table 11]**

| Protein Name | Target Protein Concentration [µM] | eSrtA Concentration [µM] | Biotin Linker Concentration [mM] |
|---|---|---|---|
| KRAS-NVOC-biotin | 3 | 20* | 0.1 |
| KRAS-NVOC2-biotin | 3 | 20* | 0.1 |
| KRAS-NVOC3-biotin | 3 | 20* | 0.1 |
| NusA-NVOC3-biotin | 40 | 3 | 0.25 |
| NusA-biotin | 40 | 3 | 0.25 |
| GST-NVOC-biotin | 40 | 1 | 1 |
| GST-NVOC2-biotin | 40 | 1 | 1 |
| GST-NVOC3-biotin | 40 | 1 | 1 |
| GST-NVOC4-biotin | 40 | 1 | 1 |
| GST-biotin | 40 | 3 | 0.5 |
| sfGFP-NVOC-biotin | 40 | 1 | 1 |
| sfGFP-NVOC2-biotin | 40 | 1 | 1 |
| sfGFP-NVOC3-biotin | 40 | 3 | 1 |
| sfGFP-NVOC4-biotin | 40 | 3 | 1 |
| sfGFP-biotin | 40 | 1 | 1 |
| GST-CMRN3-biotin | 40 | 1 | 1 |

| | | | |
|---|---|---|---|
| * Using Sepharose-immobilized eSrtA | | | |

### Preparation of KRAS-NVOC-biotin (Example 2-1), KRAS-NVOC2-biotin (Example 2-2), and KRAS-NVOC3-biotin (Example 2-3)

In reaction buffer (0.3 M Tris-HCl (pH 7.5), 0.15 M NaCl, 10 mM CaCh), KRAS4Bwt (C_FLAG-Sor-His) (3 µM), 0.1 mM biotin linker (G3-NVOC-biotin, G3-NVOC2-biotin, or G3-NVOC3-biotin), and Sepharose-immobilized eSrtA (20 µM) were mixed on ice, and the mixture was shaken in a 37°C thermostatic chamber for 2 hours. After cooling the reaction solution on ice, the supernatant was collected by centrifuging at 4°C, 280 G for 5 minutes. The collected supernatant was diluted with conservation buffer (50 mM Tris-HCl (pH 8.0), 150 mM NaCl, 10% glycerol), and buffer exchange was repeated five times using a centrifugal filter (Amicon Ultra 10K) to remove the biotin linker and obtain the target protein with the biotin linker of interest.

### Preparation of NusA-NVOC3-biotin (Example 2-4) and NusA-biotin (Comparative Example 2-1)

In reaction buffer (0.05 M Tris-HCl (pH 7.5), 0.15 M NaCl, 10 mM CaCl₂), a sortase-tagged target protein (NusA, 40 µM), a biotin linker (0.25 mM), and eSrtA (3 µM) were mixed on ice, and the mixture was incubated at 37°C for 2 hours. After the reaction, EDTA was added to obtain a final concentration of 20 mM, and the mixed solution was purified by separating the target protein from eSrtA and the biotin linker using a cOmplete^{™} His-Tag Purification Column coupled with a Superdex 75 10/300 column. The buffer composition used was 50 mM Tris-HCl (pH 8.0), 150 mM NaCl, 1 mM DTT, and 10% glycerol. After purification by column chromatography, the target protein with the biotin linker of interest was obtained by concentrating using a centrifugal filter (Amicon Ultra 3K).

### Preparation of GST-NVOC-biotin (Example 2-5) and GST-NVOC2-biotin (Example 2-6)

These were prepared by the same method as in Example 2-4, except that the target protein was GST, the concentration of the biotin linker was 1 mM, and the concentration of eSrtA was 1 µM.

### Preparation of GST-NVOC3-biotin (Example 2-7) and GST-NVOC4-biotin (Example 2-8)

These were prepared by the same method as in Example 2-4, except that the target protein was GST, the concentration of the biotin linker was 1 mM, the concentration of eSrtA was 1 µM, and a column consisting of HisTrap^{™} HP coupled with Superdex 75 10/300 was used for purification.

### Preparation of GST-biotin (Comparative Example 2-2)

This was prepared by the same method as in Example 2-4, except that the target protein was GST and the concentration of the biotin linker was 0.5 mM.

### Preparation of sfGFP-NVOC-biotin (Example 2-9), sfGFP-NVOC2-biotin (Example 2-10), and sfGFP-biotin (Comparative Example 2-3)

These were prepared by the same method as in Example 2-4, except that the target protein was GST, the concentration of the biotin linker was 1 mM, and the concentration of eSrtA was 1 µM.

### Preparation of sfGFP-NVOC3-biotin (Example 2-11) and sfGFP-NVOC4-biotin (Example 2-12)

These were prepared by the same method as in Example 2-4, except that the target protein was sfGFP, the concentration of the biotin linker was 1 mM, and a column consisting of HisTrap^{™} HP coupled with Superdex 75 10/300 was used for purification.

### Preparation of GST-CMRN3-biotin (Example 2-13)

In reaction buffer (0.05 M Tris-HCl (pH 7.5), 0.15 M NaCl, 10 mM CaCl₂), a sortase-tagged target protein (GST, 40 µM), 1 mM biotin linker (G3-CMRN3-biotin), and eSrtA (1 µM) were mixed on ice, and the mixture was incubated at 37°C for 2 hours. After the reaction, EDTA was added to obtain a final concentration of 20 mM, and the mixed solution was purified by separating the target protein from eSrtA and the biotin linker using a column consisting of HisTrap^{™} HP coupled with Superdex 75 10/300. The buffer composition used was 50 mM Tris-HCl (pH 8.0), 150 mM NaCl, 1 mM DTT, and 10% glycerol. After purification by column chromatography, the target protein with biotin linker of interest was obtained by concentrating using a centrifugal filter (Amicon Ultra 10K).

### Evaluation Example 1: Evaluation of biotin linker cleavage efficiency by UV irradiation

The photocleavage efficiency in solution of target proteins with biotin linkers was evaluated by SDS-PAGE or capillary electrophoresis. UV-LED365 manufactured by Mario network or UV-LED irradiation box MUB-031 manufactured on contract by MLC was used as the UV irradiation device, and the samples placed on ice were irradiated with light for 120 seconds at a UV wavelength of 365 nm and an illuminance of 380 to 400 mW/cm².

### Evaluation Example 1-1: Photocleavage experiment on KRAS-NVOC-biotin, KRAS-NVOC2-biotin, and KRAS-NVOC3-biotin

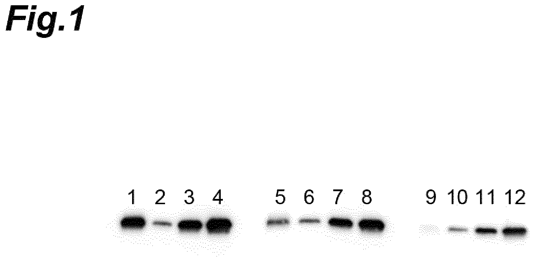

The target protein with biotin linker was diluted with 10x Ex Taq Buffer (Takara Bio) diluted 10-fold to a final concentration of 0.4 µM. The biotin linker was cleaved by irradiating with UV light 10 µL of the diluted protein solution on ice for 120 seconds. After UV irradiation, the proteins with uncleaved biotin linker were quantitatively evaluated by SDS-PAGE and Western blotting. The results of Western blotting are shown in Figure 1 and the results of the quantitative evaluation are shown in Table 12. Pierce^{™} High Sensitivity Streptavidin-HRP was used for detection.

**[Table 12]**

| **Lane** | **Target Protein** | **Sample Name** | **Uncleaved Ratio** |
|---|---|---|---|
| 1 | KRAS-NVOC-biotin | 120 s Light Irradiation | 25% |
| 2 | | Standard Sample 5% | - |
| 3 | | Standard Sample 25% | - |
| 4 | | Standard Sample 50% | - |
| 5 | KRAS-NVOC2-biotin | 120 s Light Irradiation | 6% |
| 6 | | Standard Sample 5% | - |
| 7 | | Standard Sample 25% | - |
| 8 | | Standard Sample 50% | - |
| 9 | KRAS-NVOC3-biotin | 120 s Light Irradiation | <5% (outside calibration curve) |
| 10 | | Standard Sample 5% | - |
| 11 | | Standard Sample 25% | - |
| 12 | | Standard Sample 50% | - |

### Evaluation Example 1-2: Photocleavage experiment on GST-NVOC-biotin, GST-NVOC2-biotin, GST-NVOC3-biotin, and GST-NVOC4-biotin

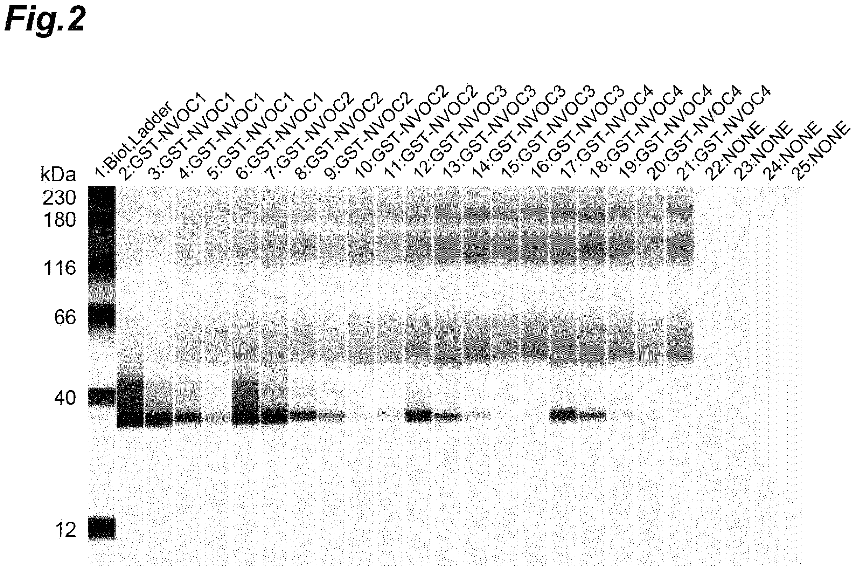

The target protein with biotin linker was diluted with dilution buffer (1X TBS, 2 mg/L BSA) to a final concentration of 0.4 µM. The biotin linker was cleaved by irradiating with UV light 10 µL of the diluted protein solution on ice for 120 seconds. After UV irradiation, the proteins with uncleaved biotin linker were quantitatively evaluated by capillary electrophoresis Jess (ProteinSimple). The results of capillary electrophoresis are shown in Figure 2 and the results of the quantitative evaluation are shown in Table 13. Pierce^{™} High Sensitivity Streptavidin-HRP was used for detection.

**[Table 13]**

| **Lane** | **Target Protein** | **Sample Name** | **Uncleaved Ratio** |
|---|---|---|---|
| 1 | Biot, Ladder*¹ | - | - |
| 2 | GST-NVOC-biotin | Standard Sample 100% | - |
| 3 | | Standard Sample 50% | - |
| 4 | | Standard Sample 25% | - |
| 5 | | Standard Sample 10% | - |
| 6 | | 120 s Light Irradiation | 53% |
| 7 | GST-NVOC2-biotin | Standard Sample 100% | - |
| 8 | | Standard Sample 50% | - |
| 9 | | Standard Sample 25% | - |
| 10 | | Standard Sample 10% | - |
| 11 | | 120 s Light Irradiation | 14% |
| 12 | GST-NVOC3-biotin | Standard Sample 100% | - |
| 13 | | Standard Sample 50% | - |
| 14 | | Standard Sample 25% | - |
| 15 | | Standard Sample 10% | - |
| 16 | | 120 s Light Irradiation | <10% (outside calibration curve) |
| 17 | GST-NVOC4-biotin | Standard Sample 100% | - |
| 18 | | Standard Sample 50% | - |
| 19 | | Standard Sample 25% | - |
| 20 | | Standard Sample 10% | - |
| 21 | | 120 s Light Irradiation | <10% (outside calibration curve) |

| | | | |
|---|---|---|---|
| *1: ProteinSimple catalog number PS-ST02EZ-8 was used as the molecular weight marker. | | | |

### Evaluation Example 1-3: Photocleavage experiment on sfGFP-NVOC-biotin, sfGFP-NVOC2-biotin, sfGFP-NVOC3-biotin, and sfGFP-NVOC4-biotin

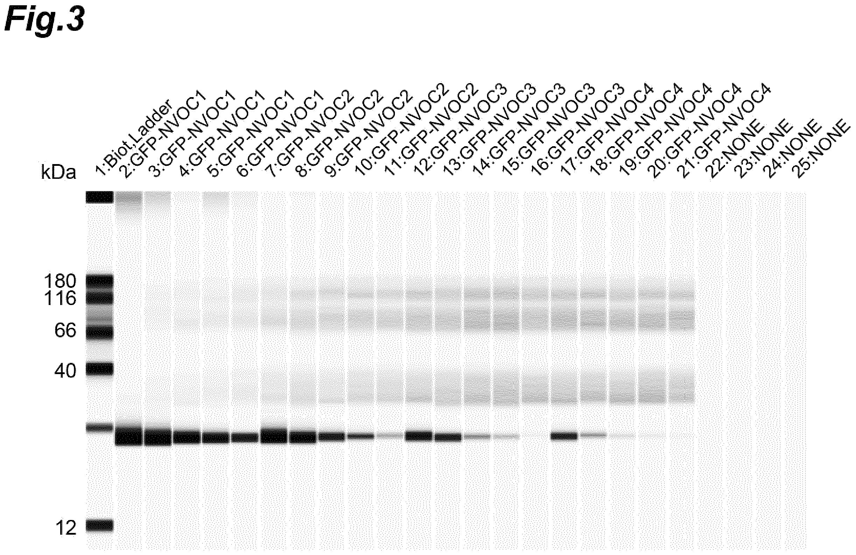

The same evaluation as in the previous section was performed. The results of capillary electrophoresis are shown in Figure 3 and the results of the quantitative evaluation are shown in Table 14.

**[Table 14]**

| **Lane** | **Target Protein** | **Sample Name** | **Uncleaved Ratio** |
|---|---|---|---|
| 1 | Biot, Ladder*¹ | - | - |
| 2 | sfGFP-NVOC-biotin | Standard Sample 100% | - |
| 3 | | Standard Sample 50% | - |
| 4 | | Standard Sample 25% | - |
| 5 | | Standard Sample 10% | - |
| 6 | | 120 s Light Irradiation | <10% (outside calibration curve) |
| 7 | sfGFP-NVOC2-biotin | Standard Sample 100% | - |
| 8 | | Standard Sample 50% | - |
| 9 | | Standard Sample 25% | - |
| 10 | | Standard Sample 10% | - |
| 11 | | 120 s Light Irradiation | <10% (outside calibration curve) |
| 12 | sfGFP-NVOC3-biotin | Standard Sample 100% | - |
| 13 | | Standard Sample 50% | - |
| 14 | | Standard Sample 25% | - |
| 15 | | Standard Sample 10% | - |
| 16 | | 120 s Light Irradiation | <10% (outside calibration curve) |
| 17 | sfGFP-NVOC4-biotin | Standard Sample 100% | - |
| 18 | | Standard Sample 50% | - |
| 19 | | Standard Sample 25% | - |
| 20 | | Standard Sample 10% | - |
| 21 | | 120 s Light Irradiation | <10% (outside calibration curve) |

| | | | |
|---|---|---|---|
| *1: ProteinSimple catalog number PS-ST02EZ-8 was used as the molecular weight marker. | | | |

### Evaluation Example 1-4: Photocleavage experiment on GST-CMRN3-biotin

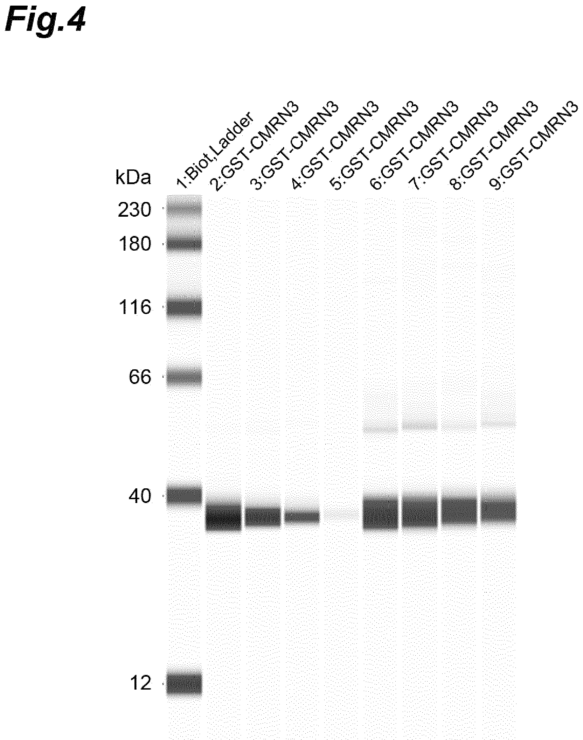

The UV irradiation time was set to 2, 5, 15, and 30 minutes, and the same evaluation as in Evaluation Example 1-2 was performed. The results of capillary electrophoresis are shown in Figure 4 and the results of the quantitative evaluation are shown in Table 15.

**[Table 15]**

| **Lane** | **Target Protein** | **Sample Name** | **Uncleaved Ratio** |
|---|---|---|---|
| 1 | Biot, Ladder*¹ | - | - |
| 2 | GST-CMRN3-biotin | Standard Sample 100% | - |
| 3 | | Standard Sample 50% | - |
| 4 | | Standard Sample 25% | - |
| 5 | | Standard Sample 10% | - |
| 6 | | 2 min Light Irradiation | 88% |
| 7 | | 5 min Light Irradiation | 80% |
| 8 | | 15 min Light Irradiation | 67% |
| 9 | | 30 min Light Irradiation | 52% |

| | | | |
|---|---|---|---|
| *1: ProteinSimple catalog number PS-ST02EZ-8 was used as the molecular weight marker. | | | |

### Evaluation Example 2: Panning of target binding peptide compounds (in vitro selection) (1) Chemical synthesis of aminoacyl-pCpA

F-Pnaz-Asp(SMe)-pCpA (pc01), an aminoacyl-pCpA used for aminoacyl-tRNA preparation, was synthesized according to the following scheme. Acbz-MeCys(StBu)-pCpA (pc02) was synthesized according to the synthesis method described in WO 2018/225864. The structure of aminoacyl-pCpA is shown in Table 16.

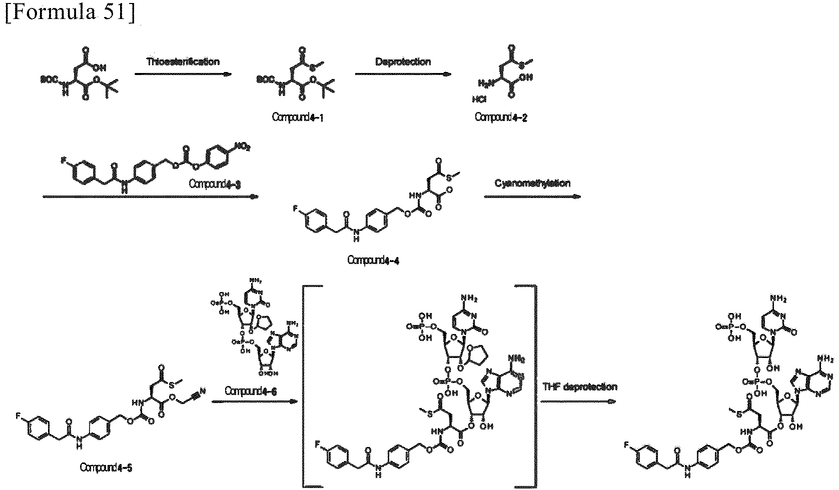

**[Table 16]**

| Aminoacyl-pCpA Name | Structure | Compound No. |
|---|---|---|
| F-Pnaz·Asp(SMe)·pCpA | 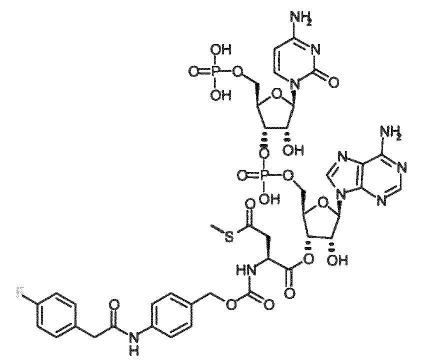 | pc01 |
| Acbz-MeCys(StBu)-pCpA | 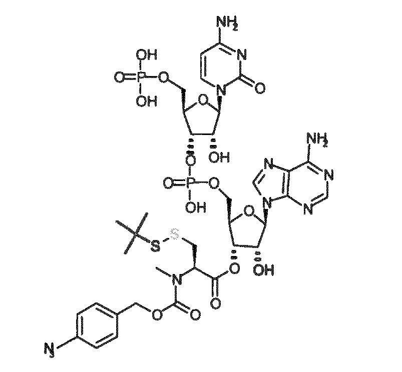 | pc02 |

### Synthesis of tert-butyl (2S)-2-[[(tert-butoxy)carbonyl]amino]-4-(methylsulfanyl)-4-oxobutanoate (Compound 4-1)

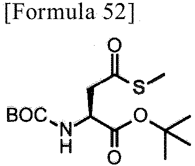

1-tert-butyl N-(tert-butoxycarbonyl)-L-aspartate (10 g, 34.56 mmol) and HOBt (5.61 g, 41.52 mmol) were dissolved in DMF, and cooled to 0°C. Then, EDC·HCl (7.97 g, 41.58 mmol) was added thereto, and the mixture was stirred for 30 minutes. Sodium thiomethoxide (2.422 g, 34.56 mmol) was added and the mixture was stirred at 0°C for 1 hour. After adding ethyl acetate and water to the reaction solution to perform extraction and washing the organic layer with saturated saline, the organic layer was dried over anhydrous sodium sulfate and concentrated under reduced pressure, and the obtained residue was used in the next step without purification.

### Synthesis of (2S)-2-amino-4-(methylsulfanyl)-4-oxobutanoic acid hydrochloride (Compound 4-2)

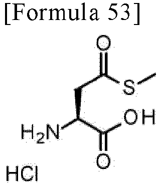

To the crude product obtained in the previous step (Compound 4-1, 12.5 g, 39.13 mmol) was added a 4M hydrochloric acid dioxane solution (380 mL) and the mixture was stirred at 70°C for 3 hours under nitrogen atmosphere. The precipitated solid was filtered and washed three times with diethyl ether, and the obtained residue was used in the next step without purification.

### Synthesis of (2S)-2-[[([4-[2-(4-fluorophenyl)acetamido]phenyl]methoxy)carbonyl]amino]-4-(methylsulfanyl)-4-oxobutanoate (Compound 4-4)

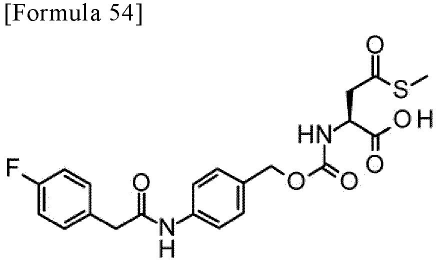

To a mixture of the crude product obtained in the previous step (Compound 4-2, 2.35 g, 11.77 mmol) and carbonic acid-(4-nitrophenyl)-4-(2-(4-fluorophenyl)acetamido)benzyl (Compound 4-3, 5.0 g, 11.78 mmol) synthesized by the method described in the Patent Literature (WO2018/143145) was added DMSO (3.9 mL), then triethylamine (357.5 mg, 3.53 mmol) was added dropwise at 0°C. The reaction mixture was stirred at room temperature for 2.5 hours, acidified by adding formic acid dropwise, and then purified by reverse-phase silica gel column chromatography (0.1% aqueous formic acid solution/0.1% solution of formic acid in acetonitrile) to obtain the title compound (Compound 4-4, 3.3 g, 62%).
LCMS (ESI) m/z = 449.3 (M+H)⁺
Retention time: 0.95 minutes (analysis condition SMDFA10)

### Synthesis of cyanomethyl (2S)-2-[[([4-[2-(4-fluorophenyl)acetamido]phenyl]methoxy)carbonyl]amino]-4-(methylsulfanyl)-4-oxobutanoate (Compound 4-5)

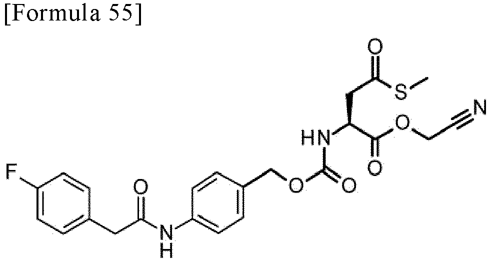

The Compound 4-4 (3.3 g, 7.36 mmol) obtained in the previous step and 2-bromoacetonitrile (15.94 g, 132.89 mmol) were dissolved in DMF (22.3 mL), then DIPEA (0.95 g, 7.35 mmol) was added dropwise at 0°C. The reaction mixture was stirred at room temperature for 45 minutes, and the reaction solution was directly purified by reverse-phase silica gel column chromatography (0.1% aqueous formic acid solution/0.1% solution of formic acid in acetonitrile). Further purification was performed by supercritical fluid chromatography to obtain the title compound (Compound 4-5, 500.1 mg, 14%).
LCMS (ESI) m/z = 486.1 (M+H)⁻
Retention time: 0.73 minutes (analysis condition SQDFA05)

### Synthesis of (2R,3S,4R,SR)-2-((((((2R,3S,4R,SR)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-((((4-(2-(4-fluorophenyl)acetamido)benzyl)oxy)carbonyl)amino)-4-(methylthio)-4-oxobutanoate (Compound No. pc01, F-Pnaz-Asp(SMe)-pCpA)

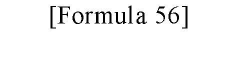

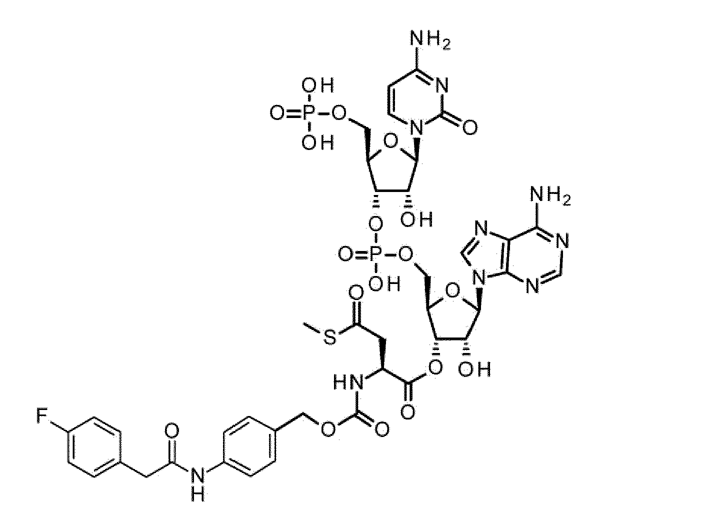

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound 4-6) (474.0 mg, 0.656 mmol) synthesized by the method described in the Literature (Helv. Chim. Acta, 90,297-310) was dissolved in buffer solution A (200 ml), then an acetonitrile solution (10 ml) of the Compound 4-5 (160.0 mg, 0.328 mmol) obtained in the previous step was injected, and the mixture was stirred at room temperature for 30 minutes. After cooling the reaction solution to 0°C, trifluoroacetic acid (10 mL) was added. After stirring the reaction solution at room temperature for 30 minutes, the reaction solution was purified by reverse-phase silica gel column chromatography (0.05% aqueous trifluoroacetic acid solution/0.05% solution of trifluoroacetic acid in acetonitrile) to obtain the title compound (Compound pc01, F-Pnaz-Asp(SMe)-pCpA) (140.0 mg, 39.4%).
LCMS (ESI) m/z = 1083.7 (M+H)⁺
Retention time: 0.49 minutes (analysis condition SQDFA05)

The buffer solution A was prepared as follows. Acetic acid was added to an aqueous solution of N,N,N-trimethylhexadecan-1-aminium chloride (6,40 g, 20 mmol) and imidazole (6.81 g, 100 mmol) to obtain a buffer solution A (1 L) of pH 8, 20 mM N,N,N-trimethylhexadecan-1-aminium, and 100 mM imidazole.

### (2) Synthesis of aminoacyl-tRNA

The aminoacyl-tRNAs used for panning and cloning assays were prepared by the method described in the Patent Literature (WO2013/100132). The nucleotide sequences of the tRNAs (CA-deficient) used are shown in Table 17 and Sequence Nos. 6 and 7. The aminoacylated tRNAs shown in Table 18 were prepared, and when used for translation, the translation solutions were prepared at the final concentrations shown in the same table. After the ligation reaction, the aminoacylated tRNAs were subjected to the operations of phenol extraction and beyond.

**[Table 17]**

| **RNA Name** | **RNA Sequence No.** | **RNA Sequence** |
|---|---|---|
| tRNAGluCUA (CA-defficient) | T-1 | GUCCCCUUCGUCUAGAGGCCCAGGACACCGCCCU cuaACGGCGGUAACAGGGGUUCGAAUCCCCUAGGGGAC GC |
| tRNAfMetCAU (CA-defficient) | T-2 | GGCGGGGUGGAGCAGCCUGGUAGCUCGUCGGGCU cauAACCCGAAGAUCGUCGGUUCAAAUCCGGCCCCCGCA AC |

**[Table 18]**

| **Name of Acylated tRNA** | **Final Concentration at Translation [µM]** |
|---|---|
| F-Pnaz-Asp(SMe)-tRNAGluCUA | 10 |
| Acbz-MeCys(StBu)-tRNAfMetCAU | 25 |

### (3) Randomized double-stranded DNA library encoding peptide compound library

The DNA library was constructed using the method described in the Patent Literature (WO2013/100132). A library where any of the codons TTT, TAC, TGG, CTG, CCG, CAT, CAG, ATT, ACG, AAC, AAA, AGT, AGG, GTG, GCT, GAC, GAA, and GGG randomly appears 8 or 9 times was prepared.

### (4) Translation solution used for panning

The translation solution used consists of the following composition. 1 mM GTP, 1 mM ATP, 20 mM phosphocreatine, 50 mM HEPES-KOH pH 7.6, 100 mM potassium acetate, 10 mM magnesium acetate, 2 mM spermidine, 1 mM dithiothreitol, 1 mg/ml *E*. *coli* MRE 600 (RNase-negative) derived tRNA (Roche) (some tRNAs are removed by the method described in the Non Patent Literature: (Yokogawa T, Kitamura Y, Nakamura D, Ohno S, Nishikawa K. 2010. Nucleic acids research 38:e89), 4 µg/ml creatine kinase, 3 µg/ml myokinase, 2 units/ml inorganic pyrophosphatase, 1.1 µg/ml nucleoside diphosphate kinase, 0.4 units/µl ribonuclease inhibitor, 0.26 µM EF-G, 2.7 µM IF1, 0.4 µM IF2, 1.5 µM IF3, 40 µM EF-Tu, 46 µM EF-Ts, 1 µM EF-P, 1.2 µM ribosome, 0.73 µM AlaRS, 0.13 µM AspRS, 0.23 µM GluRS, 0.68 µM PheRS, 0.09 µM GlyRS, 0.02 µM HisRS, 0.4 µM IleRS, 0.11 µM LysRS, 0.04 µM LeuRS, 0.38 µM AsnRS, 0.16 µM ProRS, 0.06 µM GlnRS, 0.03 µM ArgRS, 0.04 µM SerRS, 0.09 µM ThrRS, 0.02 µM ValRS, 0.03 µM TrpRS, 0.02 µM TyrRS, 3 µM *in vitro* transcribed *E. coli* tRNA Ala1B, 250 µM alanine, 250 µM aspartic acid, 250 µM glutamic acid, 250 µM phenylalanine, 250 µM glycine, 250 µM histidine, 250 µM isoleucine, 250 µM lysine, 250 µM leucine, 250 µM asparagine, 250 µM proline, 250 µM glutamine, 250 µM arginine, 250 µM serine, 250 µM threonine, 250 µM valine, 250 µM tryptophan, 250 µM tyrosine, 10 µM F-Pnaz-Asp(SMe)-tRNAGluCUA, 25 µM Acbz-MeCys(StBu)-tRNAfMetCAU, and 0.5 µM Penicillin G Amidase (PGA).

### (5) Panning

Panning was performed according to the Patent Literature (WO2013/100132) using the double-stranded DNA library and translation solution described above. After allowing the peptide library to interact with the target protein with biotin linker, it was collected with streptavidin-immobilized magnetic beads, followed by washing operations and elution operations. The targets and elution methods used for panning are listed in Table 19 below. In photoelution, the target protein and display molecule bound thereto were recovered by irradiating with UV light at a wavelength of 365 nm at an illuminance of 380 to 400 mW/cm² for 120 seconds on ice. In heat elution, the DNA molecules were recovered by incubating at 95°C for 10 minutes. In TEV elution, the target protein and display molecule bound thereto were recovered by adding TEV protease and incubating at 4°C for 10 minutes. The DNA molecules in the collected supernatant were amplified by PCR.

**[Table 19]**

| Target | Target Protein Name | Elution Method |
|---|---|---|
| GST | GST-NVOC3-biotin | Light |
| | GST-biotin | Heat |
| sfGFP | sfGFP-NVOC3-biotin | Light |
| | sfGFP-biotin | Heat |
| NusA | NusA-NVOC3-biotin | Light |
| | NusA-biotin | Heat |
| KRAS | KRAS-NVOC3-biotin | Light |
| | KRAS-TEV-biotin | TEV |

### (6) Comparison of library recovery ratios

The number of original DNA molecules used in the third round of panning and the number of recovered DNA molecules with and without the addition of the target were calculated by quantitative PCR. The percentages of the number of recovered DNA molecules with and without the addition of the target, to the number of original DNA molecules are shown in Table 20 below.

The S/N ratio was calculated by the following formula.S/N ratio = number of recovered DNA molecules with addition of target/number of recovered DNA molecules without addition of target

**[Table 20]**

| Target | Elution Method | S/N Ratio |
|---|---|---|
| GST | Light | 507 |
| | Heat | 349 |
| sfGFP | Light | 471 |
| | Heat | 7 |
| NusA | Light | 326 |
| | Heat | 57 |
| KRAS | Light | 170 |
| | TEV | 16 |

As can be seen from the table, for each target protein, a large difference in the S/N ratio was observed between photoelution and heat elution after the third round, and the S/N ratio was improved when photoelution was used compared to when heat elution was used.

### (7) Analysis of enriched sequences

Nucleotide sequence analysis of DNA pools from each round of panning was performed to construct a phylogenetic tree by UPGMA. A similarity matrix was used that assigns 1 when amino acids match and 0 when they do not match. The clusters were divided with the pruning line as 0.15 from the leaves. The total appearance frequency of the top three clusters with the highest enrichment ratio with and without the addition of the target in each round is shown in Table 21. The clusters enriched by photoelution and heat elution were similar.

**Table 21**

| Target | No. of Rounds | Elution Method | Enrichment Ratio |
|---|---|---|---|
| GST | 4 | Light | 57% |
| | | Heat | 49% |
| sfGFP | 2 | Light | 70% |
| | | Heat | 27% |
| NusA | 3 | Light | 83% |
| | | Heat | 5% |
| KRAS | 3 | Light | 94% |
| | | TEV | 77% |

As can be seen from the table, the total appearance frequency of the top three clusters with the highest enrichment ratio was improved when photoelution was used compared to when heat elution or TEV elution was used, after the second round for sfGFP, after the third round for NusA and KRAS, and after the fourth round for GST.

As can be seen from the table, the recovery ratio of the beads binder sequence was suppressed both from round 2 to round 3 and from round 3 to round 4 when photoelution was used, compared to when heat elution was used.

[Sequence Listing]

## Claims

1. A compound, having:
(i) a photocleavable moiety; and
(ii) at least one selected from the group consisting of a target substance binding site and an anchor binding site.

2. The compound according to claim 1, wherein the target substance binding site is an enzyme recognition site.

3. The compound according to claim 1 or 2, having a plurality of the photocleavable moieties.

4. The compound according to any one of claims 1 to 3, wherein the target substance binding site is recognized by at least one selected from the group consisting of ligases and proteases.

5. The compound according to any one of claims 1 to 4, wherein the absorption wavelength at which the photocleavable moiety undergoes cleavage is in the range of 300 nm or more and 500 nm or less.

6. The compound according to any one of claims 1 to 5, wherein the photocleavable moiety has a nitroveratryloxycarbonyl residue or a coumarin residue.

7. The compound according to any one of claims 1 to 6, wherein the photocleavable moiety has a structure represented by the following formula (1): 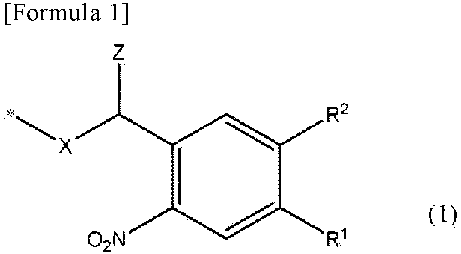 wherein
X represents O or NH;
Z represents a hydrogen atom or a C1-6 alkyl group;
R¹ represents -O-Lⁿ-L¹²-*, a C1-10 alkoxy group, or a hydrogen atom;
R² represents -O-L¹¹-L¹²-* or a C1-10 alkoxy group;
L¹¹ represents a C1-10 alkylene group;
L¹² represents a divalent group selected from the group consisting of a single bond, a C1-10 alkylene group, an amide bond, an ether bond, and combinations thereof;
any one of R¹ and R² is -O-Lⁿ-L¹²-*; and
* represents a bond.

8. The compound according to any one of claims 1 to 7, wherein the photocleavable moiety has a structure represented by the following formula (2): 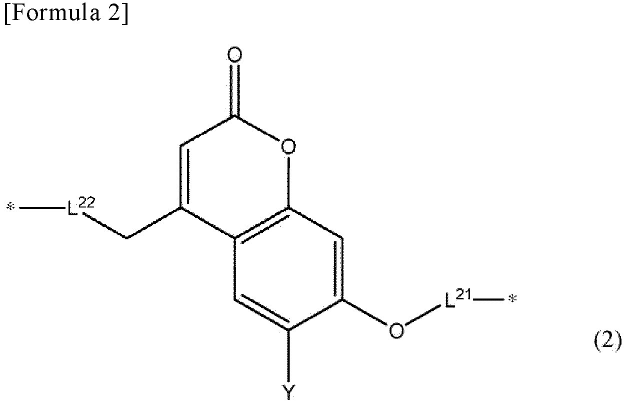 wherein
Y represents a hydrogen atom or halogen atom;
L²¹ and L²² each independently represent a divalent group selected from the group consisting of a single bond, a C1-10 alkylene group, an amide bond, an ether bond, and combinations thereof; and
* represents a bond.

9. The compound according to any one of claims 1 to 8, having a plurality of the photocleavable moieties and a spacer arm, and
wherein some or all of the plurality of photocleavable moieties are bound via the spacer arm.

10. The compound according to claim 9, wherein the spacer arm comprises an oxyethylene group, an amino acid residue, or a substituted or unsubstituted alkylene group.

11. The compound according to any one of claims 1 to 10, wherein the anchor binding site is a biotin tag.

12. A method for selecting a substance of interest capable of binding to a target substance, comprising:
a contacting step of bringing a library of object substances into contact with a conjugate comprising a compound having a photocleavable moiety and the target substance linked to the compound, and
an elution step of irradiating the conjugate with light to obtain the substance of interest.

13. The method according to claim 12, wherein the wavelength of the light is 300 nm or more and 500 nm or less.

14. The method according to claim 12 or 13, wherein the illuminance of the light is 10 mW/cm² or more and 600 mW/cm² or less.

15. The method according to any one of claims 12 to 14, wherein the irradiation time of the light is 0.5 minutes or more and 60 minutes or less.
